# EUROPEAN PATENT APPLICATION

(11) **EP 1 661 991 A1**
(43) Date of publication of application: **31.05.2006**
(21) Application number: 04772381.2
(22) Date of filing: 23.08.2004
(51) Int. Cl.: C12N 15/09, C12N 15/12, C12Q 1/68, G01N 33/50, G01N 33/15

(54) **HEPATOCELLULAR CANCER-ASSOCIATED GENE**

(30) Priority: 24.08.2003 JP 2003299363; 25.09.2003 JP 2003334003
(71) Applicant: Nihon University, Tokyo 102-8275 (JP); Nippon Flour Mills Co., Ltd, Tokyo 151-0051 (JP)
(72) Inventor: ESUMI, Mariko, c/o Nihon University, Tokyo 1028275 (JP); TAKAYAMA, Tadatoshi, c/o Nihon University, Tokyo 1028275 (JP); TAKAGI, Keiko, c/o Nihon University, Tokyo 1028275 (JP)
(74) Representative: Denison, Christopher Marcus
(86) International application number: PCT/JP2004/012425
(87) International publication number: WO 2005/021745

(57) **Abstract**

The present invention provides a method for evaluating cancer, which comprises the following steps of:
(a) collecting total RNA from an analyte;
(b) measuring the expression level of at least one gene selected from among the genes shown in Tables 1 to 8; and
(c) evaluating cancer using the measurement result as an indicator.

SEQUENCE LISTING

<110> Nihon University

<120> HEPATOCELLULAR CARCINOMA-ASSOCIATED GENE

<130> G06-0008

<150> JP 2003-299363
<151> 2003-08-22

<150> JP 2003-334444
<151> 2003-09-25

<160> 228

<170> PatentIn version 3.2

<210> 1
<211> 20
<212> DNA
<213> Artificial

<220>
<223> synthetic DNA

<400> 1
agactgtcag tactgggagc 20

<210> 2
<211> 20
<212> DNA
<213> Artificial

<220>
<223> synthetic DNA

<400> 2
gtccaggacc cttcttatcc 20

<210> 3
<211> 20
<212> DNA
<213> Artificial

<220>
<223> synthetic DNA

<400> 3
gacgtgggaa gacgtttcca 20

<210> 4
<211> 20
<212> DNA
<213> Artificial

<220>
<223> synthetic DNA

<400> 4
tggatgatgc ccgtctcctt 20

<210> 5
<211> 20
<212> DNA
<213> Artificial

<220>
<223> synthetic DNA

<400> 5
aattgcccag ggatgaggca 20

<210> 6
<211> 20
<212> DNA
<213> Artificial

<220>
<223> synthetic DNA

<400> 6
tggactcctg gatcttcctc 20

<210> 7
<211> 20
<212> DNA
<213> Artificial

<220>
<223> synthetic DNA

<400> 7
gagaactcag ctgcagtgca 20

<210> 8
<211> 20
<212> DNA
<213> Artificial

<220>
<223> synthetic DNA

<400> 8
ttctagctgg gccgctaact 20

<210> 9
<211> 20
<212> DNA
<213> Artificial

<220>
<223> synthetic DNA

<400> 9

gacgtgcaga aatggcacct                                                    20

<210>  10
<211>  20
<212>  DNA
<213>  Artificial

<220>
<223>  synthetic DNA

<400>  10
cagtcacacg gcagatggtt                                                    20

<210>  11
<211>  20
<212>  DNA
<213>  Artificial

<220>
<223>  synthetic DNA

<400>  11
cctgcatcag caccaaccaa                                                    20

<210>  12
<211>  20
<212>  DNA
<213>  Artificial

<220>
<223>  synthetic DNA

<400>  12
tggctgacct gtttctccca                                                    20

<210>  13
<211>  20

<212> DNA
<213> Artificial

<220>
<223> synthetic DNA

<400> 13
ccacatccac cactagacac 20


<210> 14
<211> 20
<212> DNA
<213> Artificial

<220>
<223> synthetic DNA

<400> 14
tgacagatgt cctctgaggc 20


<210> 15
<211> 20
<212> DNA
<213> Artificial

<220>
<223> synthetic DNA

<400> 15
cctcttcacc aggtatcctg 20


<210> 16
<211> 20
<212> DNA
<213> Artificial

<220>
<223> synthetic DNA

<400> 16
ccacagtgtc cttgggaatg 20

<210> 17
<211> 20
<212> DNA
<213> Artificial

<220>
<223> synthetic DNA

<400> 17
gctgaagcag atgcaggaca 20

<210> 18
<211> 20
<212> DNA
<213> Artificial

<220>
<223> synthetic DNA

<400> 18
ctaacgagct gacggagttg 20

<210> 19
<211> 20
<212> DNA
<213> Artificial

<220>
<223> synthetic DNA

<400> 19
ggaggttcga agacgatcag 20

<210> 20
<211> 20
<212> DNA
<213> Artificial

<220>
<223> synthetic DNA

<400> 20
gtggtgccct tccgtcaatt 20

<210> 21
<211> 20
<212> DNA
<213> Artificial

<220>
<223> synthetic DNA

<400> 21
agactgtcag tactgggagc 20

<210> 22
<211> 20
<212> DNA
<213> Artificial

<220>
<223> synthetic DNA

<400> 22
gtccaggacc cttcttatcc 20

<210> 23
<211> 20
<212> DNA
<213> Artificial

<220>
<223> synthetic DNA

<400> 23
gtgtggccaa ctgtgtcatc                                                    20


<210> 24
<211> 20
<212> DNA
<213> Artificial

<220>
<223> synthetic DNA

<400> 24
cttcagacgg tggatggagt                                                    20


<210> 25
<211> 20
<212> DNA
<213> Artificial

<220>
<223> synthetic DNA

<400> 25
aattgcccag ggatgaggca                                                    20


<210> 26
<211> 20
<212> DNA
<213> Artificial

<220>
<223> synthetic DNA

<400> 26
tggactcctg gatcttcctc                                                    20

<210> 27
<211> 20
<212> DNA
<213> Artificial

<220>
<223> synthetic DNA

<400> 27
aacaagctgg ctggaaagaa                                                    20


<210> 28
<211> 20
<212> DNA
<213> Artificial

<220>
<223> synthetic DNA

<400> 28
gtacacgaag gtgctgctca                                                    20


<210> 29
<211> 20
<212> DNA
<213> Artificial

<220>
<223> synthetic DNA

<400> 29
gacgtgcaga aatggcacct                                                    20


<210> 30
<211> 20
<212> DNA

<213>  Artificial

<220>
<223>  synthetic DNA

<400>  30
cagtcacacg gcagatggtt                                                    20

<210>  31
<211>  20
<212>  DNA
<213>  Artificial

<220>
<223>  synthetic DNA

<400>  31
cctgcatcag caccaaccaa                                                    20

<210>  32
<211>  20
<212>  DNA
<213>  Artificial

<220>
<223>  synthetic DNA

<400>  32
tggctgacct gtttctccca                                                    20

<210>  33
<211>  20
<212>  DNA
<213>  Artificial

<220>
<223>  synthetic DNA

<400> 33
tgggcaagtg aggtcttctt 20

<210> 34
<211> 20
<212> DNA
<213> Artificial

<220>
<223> synthetic DNA

<400> 34
ctgaggatca ctggtatcgc 20

<210> 35
<211> 20
<212> DNA
<213> Artificial

<220>
<223> synthetic DNA

<400> 35
gacccccagt ctcaatctca 20

<210> 36
<211> 20
<212> DNA
<213> Artificial

<220>
<223> synthetic DNA

<400> 36
agtctcttgg cgtcgtcagt 20

<210> 37

<211> 20
<212> DNA
<213> Artificial

<220>
<223> synthetic DNA

<400> 37
gctgaagcag atgcaggaca 20

<210> 38
<211> 20
<212> DNA
<213> Artificial

<220>
<223> synthetic DNA

<400> 38
ctaacgagct gacggagttg 20

<210> 39
<211> 20
<212> DNA
<213> Artificial

<220>
<223> synthetic DNA

<400> 39
ggtcggagtc aacggatttg 20

<210> 40
<211> 20
<212> DNA
<213> Artificial

<220>

<223>  synthetic DNA

<400>  40
ggatctcgct cctggaagat                                                    20



<210>  41
<211>  20
<212>  DNA
<213>  Artificial

<220>
<223>  synthetic DNA

<400>  41
caaagcatgg gcagtagctc                                                    20



<210>  42
<211>  20
<212>  DNA
<213>  Artificial

<220>
<223>  synthetic DNA

<400>  42
caagcagatc tccatggcag                                                    20



<210>  43
<211>  20
<212>  DNA
<213>  Artificial

<220>
<223>  synthetic DNA

<400>  43
tcttcaaccc cgatgtgcca                                                    20

<210> 44
<211> 20
<212> DNA
<213> Artificial

<220>
<223> synthetic DNA

<400> 44
aggctggtcg gaatggactt                                                    20


<210> 45
<211> 20
<212> DNA
<213> Artificial

<220>
<223> synthetic DNA

<400> 45
cttggaagtc tcctcttggc                                                    20


<210> 46
<211> 20
<212> DNA
<213> Artificial

<220>
<223> synthetic DNA

<400> 46
atgaacaggt cctcccgctt                                                    20


<210> 47
<211> 20
<212> DNA
<213> Artificial

<220>
<223>  synthetic DNA

<400>  47
accatcatca ccaagcgtcg                                                    20


<210>  48
<211>  20
<212>  DNA
<213>  Artificial

<220>
<223>  synthetic DNA

<400>  48
tcacctcgtc cttggtgaag                                                    20


<210>  49
<211>  20
<212>  DNA
<213>  Artificial

<220>
<223>  synthetic DNA

<400>  49
gtcgcctcac catctgtaca                                                    20


<210>  50
<211>  20
<212>  DNA
<213>  Artificial

<220>
<223>  synthetic DNA

<400>  50

ctggaggaca gctgccaata 20

<210> 51
<211> 20
<212> DNA
<213> Artificial

<220>
<223> synthetic DNA

<400> 51
tcctagaagg caaggatgcc 20

<210> 52
<211> 20
<212> DNA
<213> Artificial

<220>
<223> synthetic DNA

<400> 52
gtgggtttcc tgtccatagg 20

<210> 53
<211> 20
<212> DNA
<213> Artificial

<220>
<223> synthetic DNA

<400> 53
aacaggccat ggatctggtg 20

<210> 54
<211> 20

<212> DNA
<213> Artificial

<220>
<223> synthetic DNA

<400> 54
aggactggaa cttctccagc 20

<210> 55
<211> 20
<212> DNA
<213> Artificial

<220>
<223> synthetic DNA

<400> 55
aggataacca tgtggtggcc 20

<210> 56
<211> 20
<212> DNA
<213> Artificial

<220>
<223> synthetic DNA

<400> 56
tgcagctcct ctggcttgaa 20

<210> 57
<211> 20
<212> DNA
<213> Artificial

<220>
<223> synthetic DNA

<400> 57
gctggaactt caacagggac                                                    20

<210> 58
<211> 20
<212> DNA
<213> Artificial

<220>
<223> synthetic DNA

<400> 58
ctgaggatca ctggtatcgc                                                    20

<210> 59
<211> 20
<212> DNA
<213> Artificial

<220>
<223> synthetic DNA

<400> 59
tgaagctcaa ctgcgagcag                                                    20

<210> 60
<211> 20
<212> DNA
<213> Artificial

<220>
<223> synthetic DNA

<400> 60
acgattggct cttactgcgc                                                    20

<210> 61
<211> 20
<212> DNA
<213> Artificial

<220>
<223> synthetic DNA

<400> 61
acacagagct ctgagtcagc 20

<210> 62
<211> 20
<212> DNA
<213> Artificial

<220>
<223> synthetic DNA

<400> 62
tccaggttag gagggaagac 20

<210> 63
<211> 20
<212> DNA
<213> Artificial

<220>
<223> synthetic DNA

<400> 63
cctcaaggtc ttcctccttc 20

<210> 64
<211> 20
<212> DNA
<213> Artificial

<220>
<223> synthetic DNA

<400> 64
caccaggtac tctggtaagc 20

<210> 65
<211> 20
<212> DNA
<213> Artificial

<220>
<223> synthetic DNA

<400> 65
caaagcatgg gcagtagctc 20

<210> 66
<211> 20
<212> DNA
<213> Artificial

<220>
<223> synthetic DNA

<400> 66
caagcagatc tccatggcag 20

<210> 67
<211> 20
<212> DNA
<213> Artificial

<220>
<223> synthetic DNA

<400> 67
tcttcaaccc cgatgtgcca 20

<210> 68
<211> 20
<212> DNA
<213> Artificial

<220>
<223> synthetic DNA

<400> 68
aggctggtcg gaatggactt 20

<210> 69
<211> 20
<212> DNA
<213> Artificial

<220>
<223> synthetic DNA

<400> 69
cttggaagtc tcctcttggc 20

<210> 70
<211> 20
<212> DNA
<213> Artificial

<220>
<223> synthetic DNA

<400> 70
   atgaacaggt cctcccgctt 20

<210> 71
<211> 20
<212> DNA

<213> Artificial

<220>
<223> synthetic DNA

<400> 71
gacgtgaaga aggagccact 20

<210> 72
<211> 20
<212> DNA
<213> Artificial

<220>
<223> synthetic DNA

<400> 72
cgccatccag tacagatcct 20

<210> 73
<211> 20
<212> DNA
<213> Artificial

<220>
<223> synthetic DNA

<400> 73
   tgccatagtg gcttgatttg 20

<210> 74
<211> 20
<212> DNA
<213> Artificial

<220>
<223> synthetic DNA

<400> 74
tcagaatccc catcatcaca 20

<210> 75
<211> 20
<212> DNA
<213> Artificial

<220>
<223> synthetic DNA

<400> 75
cagggcaaag tggttcaagt 20

<210> 76
<211> 20
<212> DNA
<213> Artificial

<220>
<223> synthetic DNA

<400> 76
tctcagtggg tttcctgtcc 20

<210> 77
<211> 20
<212> DNA
<213> Artificial

<220>
<223> synthetic DNA

<400> 77
aacaggccat ggatctggtg 20

<210> 78

<211> 20
<212> DNA
<213> Artificial

<220>
<223> synthetic DNA

<400> 78
aggactggaa cttctccagc 20

<210> 79
<211> 20
<212> DNA
<213> Artificial

<220>
<223> synthetic DNA

<400> 79
aactacgcag ccttggtcag 20

<210> 80
<211> 20
<212> DNA
<213> Artificial

<220>
<223> synthetic DNA

<400> 80
tggcagttga gttgggtaaa 20

<210> 81
<211> 20
<212> DNA
<213> Artificial

<220>

<223>  synthetic DNA

<400>  81
gctggaactt caacagggac                                                    20

<210>  82
<211>  20
<212>  DNA
<213>  Artificial

<220>
<223>  synthetic DNA

<400>  82
ctgaggatca ctggtatcgc                                                    20

<210>  83
<211>  20
<212>  DNA
<213>  Artificial

<220>
<223>  synthetic DNA

<400>  83
ccactcggac agcttcttct                                                    20

<210>  84
<211>  20
<212>  DNA
<213>  Artificial

<220>
<223>  synthetic DNA

<400>  84
ggatggtctc gtggatgttc                                                    20

<210> 85
<211> 20
<212> DNA
<213> Artificial

<220>
<223> synthetic DNA

<400> 85
acacagagct ctgagtcagc 20

<210> 86
<211> 20
<212> DNA
<213> Artificial

<220>
<223> synthetic DNA

<400> 86
tccaggttag gagggaagac 20

<210> 87
<211> 20
<212> DNA
<213> Artificial

<220>
<223> synthetic DNA

<400> 87
cctcaaggtc ttcctccttc 20

<210> 88
<211> 20
<212> DNA
<213> Artificial

<220>
<223> synthetic DNA

<400> 88
caccaggtac tctggtaagc 20

<210> 89
<211> 19
<212> DNA
<213> Artificial

<220>
<223> synthetic DNA

<400> 89
ctgttggtca gccagcagt 19

<210> 90
<211> 20
<212> DNA
<213> Artificial

<220>
<223> synthetic DNA

<400> 90
gaaagccccg aagtaagacc 20

<210> 91
<211> 20
<212> DNA
<213> Artificial

<220>
<223> synthetic DNA

<400> 91

caaggaccgg ttcatttggc 20

<210> 92
<211> 20
<212> DNA
<213> Artificial

<220>
<223> synthetic DNA

<400> 92
gaacacagcc ttctcctcct 20

<210> 93
<211> 20
<212> DNA
<213> Artificial

<220>
<223> synthetic DNA

<400> 93
tgctcggggg aactatgatg 20

<210> 94
<211> 20
<212> DNA
<213> Artificial

<220>
<223> synthetic DNA

<400> 94
ggcctgtgag tctctggata 20

<210> 95
<211> 20

<212> DNA
<213> Artificial

<220>
<223> synthetic DNA

<400> 95
ggaagagtgg agagtactgg 20

<210> 96
<211> 20
<212> DNA
<213> Artificial

<220>
<223> synthetic DNA

<400> 96
atccatcggt catgctctcg 20

<210> 97
<211> 20
<212> DNA
<213> Artificial

<220>
<223> synthetic DNA

<400> 97
gtattcctgg ccctgttggt 20

<210> 98
<211> 20
<212> DNA
<213> Artificial

<220>
<223> synthetic DNA

<400> 98
ctcacccttg ttaccgctct 20

<210> 99
<211> 20
<212> DNA
<213> Artificial

<220>
<223> synthetic DNA

<400> 99
ctttgaaggg atggagctgc 20

<210> 100
<211> 20
<212> DNA
<213> Artificial

<220>
<223> synthetic DNA

<400> 100
atcgtacatg ccccttggga 20

<210> 101
<211> 20
<212> DNA
<213> Artificial

<220>
<223> synthetic DNA

<400> 101
ggcctctatc gtcaacaagg 20

<210> 102
<211> 20
<212> DNA
<213> Artificial

<220>
<223> synthetic DNA

<400> 102
gcgttgaact tgacagcaaa 20

<210> 103
<211> 20
<212> DNA
<213> Artificial

<220>
<223> synthetic DNA

<400> 103
taccaatggt gcctcctgga 20

<210> 104
<211> 20
<212> DNA
<213> Artificial

<220>
<223> synthetic DNA

<400> 104
ccacagactc tgtcaggttg 20

<210> 105
<211> 20
<212> DNA
<213> Artificial

<220>
<223> synthetic DNA

<400> 105
ctggaaaggg ccaaggagat                                                    20

<210> 106
<211> 20
<212> DNA
<213> Artificial

<220>
<223> synthetic DNA

<400> 106
tctgggtctt ggctggtttc                                                    20

<210> 107
<211> 20
<212> DNA
<213> Artificial

<220>
<223> synthetic DNA

<400> 107
tggccagaca gacatgtcga                                                    20

<210> 108
<211> 20
<212> DNA
<213> Artificial

<220>
<223> synthetic DNA

<400> 108
tcgaggacag ttccgtgtag                                                    20

<210> 109
<211> 20
<212> DNA
<213> Artificial

<220>
<223> synthetic DNA

<400> 109
tctctggagg ctggagaaag 20

<210> 110
<211> 20
<212> DNA
<213> Artificial

<220>
<223> synthetic DNA

<400> 110
gtttccagct tcacagccca 20

<210> 111
<211> 20
<212> DNA
<213> Artificial

<220>
<223> synthetic DNA

<400> 111
attggctcga agctgcagga 20

<210> 112
<211> 20
<212> DNA

<213> Artificial

<220>
<223> synthetic DNA

<400> 112
ggaaggtgac atactcctgg 20

<210> 113
<211> 20
<212> DNA
<213> Artificial

<220>
<223> synthetic DNA

<400> 113
tactccaatg caaccaccaa 20

<210> 114
<211> 20
<212> DNA
<213> Artificial

<220>
<223> synthetic DNA

<400> 114
aacacaagtt gggatgcaca 20

<210> 115
<211> 20
<212> DNA
<213> Artificial

<220>
<223> synthetic DNA

<400> 115
tggtgtgctg gctgtgcatt 20

<210> 116
<211> 20
<212> DNA
<213> Artificial

<220>
<223> synthetic DNA

<400> 116
gaccagatag agaacgccga 20

<210> 117
<211> 20
<212> DNA
<213> Artificial

<220>
<223> synthetic DNA

<400> 117
gtgaatgcct ctggagtggt 20

<210> 118
<211> 20
<212> DNA
<213> Artificial

<220>
<223> synthetic DNA

<400> 118
ttctgttctg acgccaagtg 20

<210> 119

<211> 20
<212> DNA
<213> Artificial

<220>
<223> synthetic DNA

<400> 119
gttctagcca gtacttccgg 20

<210> 120
<211> 19
<212> DNA
<213> Artificial

<220>
<223> synthetic DNA

<400> 120
actcgctccg aattcttgc 19

<210> 121
<211> 20
<212> DNA
<213> Artificial

<220>
<223> synthetic DNA

<400> 121
attccgacct cgtcatcagg 20

<210> 122
<211> 20
<212> DNA
<213> Artificial

<220>

<223> synthetic DNA

<400> 122
gctggtataa ggtggtctgg 20

<210> 123
<211> 20
<212> DNA
<213> Artificial

<220>
<223> synthetic DNA

<400> 123
ggactttccc aatctgccct 20

<210> 124
<211> 20
<212> DNA
<213> Artificial

<220>
<223> synthetic DNA

<400> 124
aggttgtgct tgcgggcaat 20

<210> 125
<211> 20
<212> DNA
<213> Artificial

<220>
<223> synthetic DNA

<400> 125
aggagagaag ggtgcagaag 20

<210> 126
<211> 20
<212> DNA
<213> Artificial

<220>
<223> synthetic DNA

<400> 126
ccttccatag tagccacgtc 20

<210> 127
<211> 20
<212> DNA
<213> Artificial

<220>
<223> synthetic DNA

<400> 127
acaacctgtg cggggaatca 20

<210> 128
<211> 20
<212> DNA
<213> Artificial

<220>
<223> synthetic DNA

<400> 128
ggtcatagca gagtttggcc 20

<210> 129
<211> 20
<212> DNA
<213> Artificial

<220>
<223> synthetic DNA

<400> 129
gcagaaggac aggacaaagc 20

<210> 130
<211> 20
<212> DNA
<213> Artificial

<220>
<223> synthetic DNA

<400> 130
caggctcttc ggtaaactcg 20

<210> 131
<211> 20
<212> DNA
<213> Artificial

<220>
<223> synthetic DNA

<400> 131
atggagatga tcccttgctg 20

<210> 132
<211> 20
<212> DNA
<213> Artificial

<220>
<223> synthetic DNA

<400> 132

aggtgttctg tgccttccac                                                    20

<210>  133
<211>  20
<212>  DNA
<213>  Artificial

<220>
<223>  synthetic DNA

<400>  133
gctctaagcc tgtccacgag                                                    20

<210>  134
<211>  20
<212>  DNA
<213>  Artificial

<220>
<223>  synthetic DNA

<400>  134
cgcttcctga agtagcgatt                                                    20

<210>  135
<211>  20
<212>  DNA
<213>  Artificial

<220>
<223>  synthetic DNA

<400>  135
gcgacacagg agtgtcaaga                                                    20

<210>  136
<211>  20

<212> DNA
<213> Artificial

<220>
<223> synthetic DNA

<400> 136
tgaccatgat gtagccctga 20

<210> 137
<211> 20
<212> DNA
<213> Artificial

<220>
<223> synthetic DNA

<400> 137
tgaatggcca cagtgatgtt 20

<210> 138
<211> 20
<212> DNA
<213> Artificial

<220>
<223> synthetic DNA

<400> 138
ccattccgtt tttgaaatgc 20

<210> 139
<211> 20
<212> DNA
<213> Artificial

<220>
<223> synthetic DNA

<400> 139
tcgtaagtgg ggctataccg                                                    20

<210> 140
<211> 20
<212> DNA
<213> Artificial

<220>
<223> synthetic DNA

<400> 140
ctggttgggt ctgtggaact                                                    20

<210> 141
<211> 20
<212> DNA
<213> Artificial

<220>
<223> synthetic DNA

<400> 141
ctgaccaccg ggtgtacttt                                                    20

<210> 142
<211> 20
<212> DNA
<213> Artificial

<220>
<223> synthetic DNA

<400> 142
gacaagtagg gcagcacctc                                                    20

<210> 143
<211> 20
<212> DNA
<213> Artificial

<220>
<223> synthetic DNA

<400> 143
ggaacccgta cacatggact 20

<210> 144
<211> 20
<212> DNA
<213> Artificial

<220>
<223> synthetic DNA

<400> 144
   aacgtccaat agcccttacg 20

<210> 145
<211> 20
<212> DNA
<213> Artificial

<220>
<223> synthetic DNA

<400> 145
cgcccacttc tttgagtttc 20

<210> 146
<211> 20
<212> DNA
<213> Artificial

<220>
<223> synthetic DNA

<400> 146
catgaccgtc cctatcttgc 20

<210> 147
<211> 20
<212> DNA
<213> Artificial

<220>
<223> synthetic DNA

<400> 147
tatggggtct ttgctggaag 20

<210> 148
<211> 20
<212> DNA
<213> Artificial

<220>
<223> synthetic DNA

<400> 148
gcccacgtga tgatacttga 20

<210> 149
<211> 20
<212> DNA
<213> Artificial

<220>
<223> synthetic DNA

<400> 149
ggtcacttca tgcctgtcct 20

<210> 150
<211> 20
<212> DNA
<213> Artificial

<220>
<223> synthetic DNA

<400> 150
tatggcggaa gccagcttca 20

<210> 151
<211> 20
<212> DNA
<213> Artificial

<220>
<223> synthetic DNA

<400> 151
cacaagccct ttgaatccat 20

<210> 152
<211> 20
<212> DNA
<213> Artificial

<220>
<223> synthetic DNA

<400> 152
tgtctccagc tccacacaag 20

<210> 153
<211> 21
<212> DNA

<213> Artificial

<220>
<223> synthetic DNA

<400> 153
ttgagaattc cagagccaag a 21

<210> 154
<211> 20
<212> DNA
<213> Artificial

<220>
<223> synthetic DNA

<400> 154
cacccatctt caccacacac 20

<210> 155
<211> 20
<212> DNA
<213> Artificial

<220>
<223> synthetic DNA

<400> 155
aagaccgaac tgagcaagga 20

<210> 156
<211> 20
<212> DNA
<213> Artificial

<220>
<223> synthetic DNA

<400> 156
   tttttcccag gtccacagtc                                                    20

<210> 157
<211> 20
<212> DNA
<213> Artificial

<220>
<223> synthetic DNA

<400> 157
cgggcaagac tttctttga                                                    20

<210> 158
<211> 20
<212> DNA
<213> Artificial

<220>
<223> synthetic DNA

<400> 158
tgccttcctc atccccttat                                                   20

<210> 159
<211> 20
<212> DNA
<213> Artificial

<220>
<223> synthetic DNA

<400> 159
tccaaggatg atctcccact                                                   20

<210> 160

<211> 20
<212> DNA
<213> Artificial

<220>
<223> synthetic DNA

<400> 160
agcatccgat tccttcttca                                                    20


<210> 161
<211> 20
<212> DNA
<213> Artificial

<220>
<223> synthetic DNA

<400> 161
tgataagcac gttgcaggag                                                    20


<210> 162
<211> 20
<212> DNA
<213> Artificial

<220>
<223> synthetic DNA

<400> 162
aagtcagctg ggtttccaga                                                    20


<210> 163
<211> 20
<212> DNA
<213> Artificial

<220>

<223> synthetic DNA

<400> 163
ggtgtcagag ccagttgtca 20

<210> 164
<211> 20
<212> DNA
<213> Artificial

<220>
<223> synthetic DNA

<400> 164
aaatttccac atcggcagtc 20

<210> 165
<211> 20
<212> DNA
<213> Artificial

<220>
<223> synthetic DNA

<400> 165
ccaccattgg taccatttcc 20

<210> 166
<211> 20
<212> DNA
<213> Artificial

<220>
<223> synthetic DNA

<400> 166
cccctcacct gaacctcata 20

<210> 167
<211> 22
<212> DNA
<213> Artificial

<220>
<223> synthetic DNA

<400> 167
ttttctggaa cattcaaatt ca 22

<210> 168
<211> 20
<212> DNA
<213> Artificial

<220>
<223> synthetic DNA

<400> 168
cactttttgt catcgctgga 20

<210> 169
<211> 20
<212> DNA
<213> Artificial

<220>
<223> synthetic DNA

<400> 169
tgcagtggaa tacggatcaa 20

<210> 170
<211> 20
<212> DNA
<213> Artificial

<220>
<223> synthetic DNA

<400> 170
ggaagcagac cacagaggag 20

<210> 171
<211> 20
<212> DNA
<213> Artificial

<220>
<223> synthetic DNA

<400> 171
agtggaaggt gtgggtgaag 20

<210> 172
<211> 20
<212> DNA
<213> Artificial

<220>
<223> synthetic DNA

<400> 172
caaccataca ctgccacagg 20

<210> 173
<211> 20
<212> DNA
<213> Artificial

<220>
<223> synthetic DNA

<400> 173

cacttaagga gcgctggaac                                                    20

<210>  174
<211>  20
<212>  DNA
<213>  Artificial

<220>
<223>  synthetic DNA

<400>  174
tttgcagtct ggcaagtgag                                                    20

<210>  175
<211>  21
<212>  DNA
<213>  Artificial

<220>
<223>  synthetic DNA

<400>  175
gccacttctg cttctgtgtt t                                                  21

<210>  176
<211>  22
<212>  DNA
<213>  Artificial

<220>
<223>  synthetic DNA

<400>  176
tccaccataa aagatgtgga aa                                                 22

<210>  177
<211>  20

<212>  DNA
<213>  Artificial

<220>
<223>  synthetic DNA

<400>  177
cctccactca tgtcccattt                                                    20


<210>  178
<211>  20
<212>  DNA
<213>  Artificial

<220>
<223>  synthetic DNA

<400>  178
tcaagccatg cttttctgtg                                                    20


<210>  179
<211>  20
<212>  DNA
<213>  Artificial

<220>
<223>  synthetic DNA

<400>  179
atttagcca atggcctcct                                                    20


<210>  180
<211>  20
<212>  DNA
<213>  Artificial

<220>
<223>  synthetic DNA

<400> 180
cactggtttg cagcgataga 20

<210> 181
<211> 20
<212> DNA
<213> Artificial

<220>
<223> synthetic DNA

<400> 181
attggtgaat tgggattgga 20

<210> 182
<211> 20
<212> DNA
<213> Artificial

<220>
<223> synthetic DNA

<400> 182
gaagcccacc acagtaggaa 20

<210> 183
<211> 20
<212> DNA
<213> Artificial

<220>
<223> synthetic DNA

<400> 183
tggatcgaat ccaaacacaa 20

<210> 184
<211> 20
<212> DNA
<213> Artificial

<220>
<223> synthetic DNA

<400> 184
ctggcttgtc tgcaaacctt 20

<210> 185
<211> 20
<212> DNA
<213> Artificial

<220>
<223> synthetic DNA

<400> 185
ctccatcatc cacccacttt 20

<210> 186
<211> 20
<212> DNA
<213> Artificial

<220>
<223> synthetic DNA

<400> 186
ggaaggccag caagtgtaga 20

<210> 187
<211> 20
<212> DNA
<213> Artificial

<220>
<223> synthetic DNA

<400> 187
gccagaaaat tgaccctgag 20

<210> 188
<211> 20
<212> DNA
<213> Artificial

<220>
<223> synthetic DNA

<400> 188
cagctgctca gcgatcttac 20

<210> 189
<211> 20
<212> DNA
<213> Artificial

<220>
<223> synthetic DNA

<400> 189
ccctcatcgt gtcaagtcaa 20

<210> 190
<211> 20
<212> DNA
<213> Artificial

<220>
<223> synthetic DNA

<400> 190
agcatcaaac agacccaacc 20

<210> 191
<211> 20
<212> DNA
<213> Artificial

<220>
<223> synthetic DNA

<400> 191
ttgtttggct gggatagagg 20

<210> 192
<211> 20
<212> DNA
<213> Artificial

<220>
<223> synthetic DNA

<400> 192
gctctgtccg gatagctacg 20

<210> 193
<211> 20
<212> DNA
<213> Artificial

<220>
<223> synthetic DNA

<400> 193
ccctacaagg tgaacccaga 20

<210> 194
<211> 20
<212> DNA

<213> Artificial

<220>
<223> synthetic DNA

<400> 194
ggagtagcag ctggttcctg 20

<210> 195
<211> 20
<212> DNA
<213> Artificial

<220>
<223> synthetic DNA

<400> 195
tgacaaccta tgccatttcg 20

<210> 196
<211> 21
<212> DNA
<213> Artificial

<220>
<223> synthetic DNA

<400> 196
ccacacaaga cctatgatag a 21

<210> 197
<211> 20
<212> DNA
<213> Artificial

<220>
<223> synthetic DNA

<400> 197
ctcaaggatg acgtgggttt 20

<210> 198
<211> 20
<212> DNA
<213> Artificial

<220>
<223> synthetic DNA

<400> 198
gatttcctct ggccaattca 20

<210> 199
<211> 20
<212> DNA
<213> Artificial

<220>
<223> synthetic DNA

<400> 199
aactacgcag ccttggtcag 20

<210> 200
<211> 20
<212> DNA
<213> Artificial

<220>
<223> synthetic DNA

<400> 200
tggcagttga gttgggtaaa 20

<210> 201

<211> 20
<212> DNA
<213> Artificial

<220>
<223> synthetic DNA

<400> 201
gatgttacca atcccgttcg 20

<210> 202
<211> 20
<212> DNA
<213> Artificial

<220>
<223> synthetic DNA

<400> 202
tgggctccta tatgcggtta 20

<210> 203
<211> 20
<212> DNA
<213> Artificial

<220>
<223> synthetic DNA

<400> 203
tgctttcaac gtggagtttg 20

<210> 204
<211> 20
<212> DNA
<213> Artificial

<220>

<223>  synthetic DNA

<400>  204
ccccatattt ggtgttccag                                                    20

<210>  205
<211>  20
<212>  DNA
<213>  Artificial

<220>
<223>  synthetic DNA

<400>  205
caaaatgctg ctggtgaaga                                                    20

<210>  206
<211>  20
<212>  DNA
<213>  Artificial

<220>
<223>  synthetic DNA

<400>  206
gcctctgtca gctcaaggac                                                    20

<210>  207
<211>  20
<212>  DNA
<213>  Artificial

<220>
<223>  synthetic DNA

<400>  207
gtcgtcagca gccatgttta                                                    20

<210> 208
<211> 20
<212> DNA
<213> Artificial

<220>
<223> synthetic DNA

<400> 208
ggcaggtcaa aggtcatgtt                                                    20



<210> 209
<211> 20
<212> DNA
<213> Artificial

<220>
<223> synthetic DNA

<400> 209
gggatgcttg aagatggaaa                                                    20



<210> 210
<211> 20
<212> DNA
<213> Artificial

<220>
<223> synthetic DNA

<400> 210
cagtggcacc ataggcataa                                                    20



<210> 211
<211> 20
<212> DNA
<213> Artificial

<220>
<223> synthetic DNA

<400> 211
gctcctgggt agaactgcac 20

<210> 212
<211> 20
<212> DNA
<213> Artificial

<220>
<223> synthetic DNA

<400> 212
   gccctgttgg tatcttgtgg 20

<210> 213
<211> 20
<212> DNA
<213> Artificial

<220>
<223> synthetic DNA

<400> 213
ttgaggaaat cctggacctg 20

<210> 214
<211> 20
<212> DNA
<213> Artificial

<220>
<223> synthetic DNA

<400> 214

ttgaggtctc gcaccttctt                                                    20

<210>  215
<211>  20
<212>  DNA
<213>  Artificial

<220>
<223>  synthetic DNA

<400>  215
ctgatggagt acgcaaagca                                                    20

<210>  216
<211>  20
<212>  DNA
<213>  Artificial

<220>
<223>  synthetic DNA

<400>  216
ctcgagaatg tcaggggtgt                                                    20

<210>  217
<211>  18
<212>  DNA
<213>  Artificial

<220>
<223>  synthetic DNA

<400>  217
acagagcctc gcctttgc                                                      18

<210>  218
<211>  18

<212> DNA
<213> Artificial

<220>
<223> synthetic DNA

<400> 218
cacgatggag gggaagac 18

<210> 219
<211> 20
<212> DNA
<213> Artificial

<220>
<223> synthetic DNA

<400> 219
gagctgggaa gattcgaaca 20

<210> 220
<211> 20
<212> DNA
<213> Artificial

<220>
<223> synthetic DNA

<400> 220
agagatacgc aggtgcaggt 20

<210> 221
<211> 19
<212> DNA
<213> Artificial

<220>
<223> synthetic DNA

<400> 221
gagatctcgc cggctttac 19

<210> 222
<211> 20
<212> DNA
<213> Artificial

<220>
<223> synthetic DNA

<400> 222
cgcgagagtc aaagatctcc 20

<210> 223
<211> 21
<212> DNA
<213> Artificial

<220>
<223> synthetic DNA

<400> 223
cagctccagg aaatgctagt g 21

<210> 224
<211> 20
<212> DNA
<213> Artificial

<220>
<223> synthetic DNA

<400> 224
ggtggaactt gggatcagac 20

<210> 225
<211> 20
<212> DNA
<213> Artificial

<220>
<223> synthetic DNA

<400> 225
gagggaaggc ttagccatgt 20

<210> 226
<211> 20
<212> DNA
<213> Artificial

<220>
<223> synthetic DNA

<400> 226
ttgaagggtc catgcctatc 20

<210> 227
<211> 20
<212> DNA
<213> Artificial

<220>
<223> synthetic DNA

<400> 227
gcctgtaagt acggggacaa 20

<210> 228
<211> 20
<212> DNA
<213> Artificial

<220>
<223> synthetic DNA

<400> 228
ctcttcagcg ttgtggatga 20

## Description

### TECHNICAL FIELD

The present invention relates to a gene associated with hepatocellular carcinoma, and particularly to a gene associated with the recurrence of hepatocellular carcinoma.

### BACKGROUND ART

Almost all types of hepatocellular carcinomas are developed from chronic hepatitis caused by viral hepatitis. The causal viruses thereof are hepatitis C virus and hepatitis B virus. If a patient is persistently infected with either hepatitis C virus or hepatitis B virus, there are no therapeutic methods therefor. The patient does nothing but only facing a fear of developing liver cirrhosis or hepatocellular carcinoma. Interferon has been used as an agent for treating hepatitis. However, effective examples are only 30%, and thus this is not necessarily a sufficient therapeutic agent. Under the present circumstances, there are almost no effective examples, in particular, for chronic hepatitis. Nevertheless, even if such viruses cannot be eliminated, if progression of pathologic conditions can be suppressed, it leads to prevention of liver cirrhosis or hepatocellular carcinoma. Thus, it is considered important to clarify the factor of developing pathologic conditions at a molecular level.

If once hepatocellular carcinoma has been developed, even if a surgical radical operation is made, the recurrence of cancer in the remaining liver appears at a high frequency. The survival rate obtained 5 years after the operation of liver cancer is 51 % on a national accumulation base. It has been reported that such recurrence appears at approximately 25% of cases 1 year after hepatectomy, at 50% thereof 2 years after hepatectomy, and at 80% thereof 5 years after hepatectomy. Hence, it cannot be said that remaining liver tissues are normal liver tissues, but it is considered that a bud of the recurrence of hepatocellular carcinoma has already existed. At present, it has been reported that recurrence risk factors include the maximum diameter of a tumor, the number of tumors, tumor embolus of portal vein, a preoperative AFP value, intrahepatic metastasis, the presence or absence of liver cirrhosis, etc. However, in order to develop a method for predicting and preventing the recurrence of hepatocellular carcinoma, it is necessary to find at a molecular level a factor of determining the presence or absence of recurrence, which is associated with such risk factors. Such a factor obtained at a molecular level is considered to be a factor, which is associated not only with recurrence but also with the development of hepatocellular carcinoma or progression of pathologic conditions. In recent years, as a result of gene expression analysis using a DNA microarray, it has become possible to classify more in detail such pathologic conditions based on the difference in the expression patterns of genes as a whole. To date, histological or immunological means have been mainly used for classification of cancers. However, cancers classified into the same type have different clinical courses and therapeutic effects depending on individual cases. If there were a means for classifying such cancers more in detail, it would become possible to offer treatment depending on individual cases. It is considered that the gene expression analysis using a DNA microarray constitutes a powerful method for knowing the prognosis of such cancers.

To date, the DNA microarray analysis has clarified the following points associated with hepatocellular carcinoma:
(i) the types of genes, the expressions of which are different between a tumor tissue and a nontumor tissue (Shirota Y, Kaneko S, Honda M, et al. Identification of differentially expressed gene in hepatocellular carcinoma with cDNA microarrays. Hepatology 2001; 33: 832-840, Xu X, Huang J, Xu Z, et al. Insight into hepatocellular carcinogenesis at transcriptome level by comparing gene expression profiles of hepatocellular carcinoma with those of corresponding noncancerous liver. Proc. Nat. Acad. Sci. USA. 2001; 98: 15089-15094);
(ii) in terms of the differentiation degree of cancer tissues, the types of genes, the expressions of which are different (Shirota Y, Kaneko S, Honda M, et al. Identification of differentially expressed gene in hepatocellular carcinoma with cDNA microarrays. Hepatology 2001; 33: 832-840, Okabe H, Satoh S, Kato T, et al. Genome-wide analysis of gene expression in human hepatocellular carcinomas using cDNA microarray: Identification of genes involved in viral carcinogenesis and tumor progression. Cancer res. 2001 ; 61 : 2129-2137);
(iii) the types of genes, the expressions of which are different between hepatocellular carcinoma derived from hepatitis B and hepatocellular carcinoma derived from hepatitis C (Okabe H, Satoh S, Kato T, et al. Genome-wide analysis of gene expression in human hepatocellular carcinomas using cDNA microarray: Identification of genes involved in viral carcinogenesis and tumor progression. Cancer res. 2001; 61: 2129- 2137);
(iv) the types of genes, the expressions of which are different depending on the presence or absence of vascular invasion of hepatocellular carcinoma (Okabe H, Satoh S, Kato T, et al. Genome-wide analysis of gene expression in human hepatocellular carcinomas using cDNA microarray : Identification of genes involved in viral carcinogenesis and tumor progression. Cancer res. 2001; 61: 2129- 2137); and
(v) the type of a change in gene expression observed among intrahepatic metastatic cancers, as a result of the clonal analysis of multinodular hepatocellular carcinoma (Cheung S, Chen X, Guan X, et al. Identify metastasis-associated gene in hepatocellular carcinoma through clonality delineation for multinodular tumor. Cancer res. 2002; 62: 4711- 4721).

However, with regard to genes associated with recurrence, only the analysis of Iizuka et al. on cancer tissues has existed (Iizuka N, Oka M, Yamada-Okabe H, et al. Oligonucleotide microarray for prediction of early intrahepatic recurrence of hepatocellular carcinoma after curative resection. Lancet 2003; 361: 923-929). The analysis of nontumor liver tissues, which reflects the remaining liver tissues, has not yet been achieved.

### DISCLOSURE OF THE INVENTION

It is an object of the present invention to provide a gene associated with hepatocellular carcinoma, and particularly, a gene, which predicts the recurrence of the cancer.

As a result of intensive studies directed towards achieving the aforementioned object, the present inventor has studied the profile of gene expression based on a case where hepatocellular carcinoma has recurred and a case where hepatocellular carcinoma has not recurred, and has succeeded in identification of a gene associated with hepatocellular carcinoma, thereby completing the present invention.

That is to say, the present invention has the following features:
(1) A method for evaluating cancer, which comprises the following steps of:
   (a) collecting total RNA from an analyte;
   (b) measuring the expression level of at least one gene selected from among the genes shown in Tables 1 to 8; and
   (c) evaluating cancer using the measurement result as an indicator.

In the present invention, from among the genes shown in Tables 1 to 8, at least one gene selected from the group consisting of the PSMB8 gene, the RALGDS gene, the GBP1 gene, the RPS14 gene, the CXCL9 gene, the DKFZp564F212 gene, the CYP1B1 gene, the TNFSF10 gene, the NR0B2 gene, the MAFB gene, the BF530535 gene, the MRPL24 gene, the QPRT gene, the VNN1 gene, and the IRS2 gene, can be used, for example. Otherwise, from among the genes shown in Tables 1 to 8, at least one gene selected from the group consisting of the PZP gene, the MAP3K5 gene, the TNFSF14 gene, the LMNA gene, the CYP1A1 gene, and the IGFBP3 gene, can be used, for example.

In addition, when such measurement is carried out using GAPDH as an internal standard gene, from among the genes shown in Tables 1 to 8, each gene contained in a gene set consisting of the VNN1 gene and the MRPL24 gene, or a gene set consisting of the PRODH gene, the LMNA gene, and the MAP3K12 gene, can be used.

Moreover, when such measurement is carried out using 18S rRNA as an internal standard gene, from among the genes shown in Tables 1 to 8, each gene contained in a gene set consisting of the VNN1 gene, the CXCL9 gene, the GBP1 gene, and the RALGDS gene, or a gene set consisting of the LMNA gene, the LTBP2 gene, the COLIA2 gene, and the PZP gene, can be used.

The above evaluation of cancer involves prediction of the presence or absence of metastasis or recurrence. Further, an example of such cancer is hepatocellular carcinoma.

The expression level of a gene can be measured by amplifying the gene, using at least one set of primers consisting of the nucleotide sequences shown in SEQ ID NOS: 2n-1 and 2n (wherein n represents an integer between 1 and 114). Otherwise, the expression level of a gene can be measured by amplifying the gene, using a set of primers for amplifying each gene contained in at least one gene set selected from the group consisting of a gene set consisting of the VNN1 gene and the MRPL24 gene, a gene set consisting of the PRODH gene, the LMNA gene, and the MAP3K12 gene, a gene set consisting of the VNN1 gene, the CXCL9 gene, the GBP1 gene, and the RALGDS gene, and a gene set consisting of the LMNA gene, the LTBP2 gene, the COL1A2 gene, and the PZP gene.
(2) A primer set, which comprises at least one set of primers consisting of the nucleotide sequences shown in SEQ ID NOS: 2n-1 and 2n (wherein n represents an integer between 1 and 114).
(3) A primer set, which comprises a set of primers for amplifying each gene contained in at least one gene set selected from the group consisting of a gene set consisting of the VNN 1 gene and the MRPL24 gene, a gene set consisting of the PRODH gene, the LMNA gene, and the MAP3K12 gene, a gene set consisting of the VNN1 gene, the CXCL9 gene, the GBP1 gene, and the RALGDS gene, and a gene set consisting of the LMNA gene, the LTBP2 gene, the COL1A2 gene, and the PZP gene.
(4) A kit for evaluating cancer, which comprises any gene shown in Tables 1 to 8.

An example of the aforementioned gene is at least one gene selected from the group consisting of the RALGDS gene, the GBP1 gene, the DKFZp564F212 gene, the TNFSF10 gene, and the QPRT gene.

Moreover, another example of the aforementioned gene is each gene contained in at least one gene set selected from the group consisting of a gene set consisting of the VNN1 gene and the MRPL24 gene, a gene set consisting of the PRODH gene, the LMNA gene, and the MAP3K12 gene, a gene set consisting of the VNN1 gene, the CXCL9 gene, the GBP1 gene, and the RALGDS gene, and a gene set consisting of the LMNA gene, the LTBP2 gene, the COLIA2 gene, and the PZP gene.

Furthermore, the kit of the present invention may comprise the aforementioned primer set.

The present invention provides a gene useful for predicting the recurrence of hepatocellular carcinoma. Cancer can be evaluated by analyzing the increased expression state of such a gene. In particular, using the gene of the present invention, the recurrence of hepatocellular carcinoma can be predicted, and the obtained prediction information is useful for the subsequent therapeutic strategy. Moreover, the use of such a gene and a gene product enables the development of a treatment method for preventing recurrence.

### BRIEF DESCRIPTION OF THE DRAWING

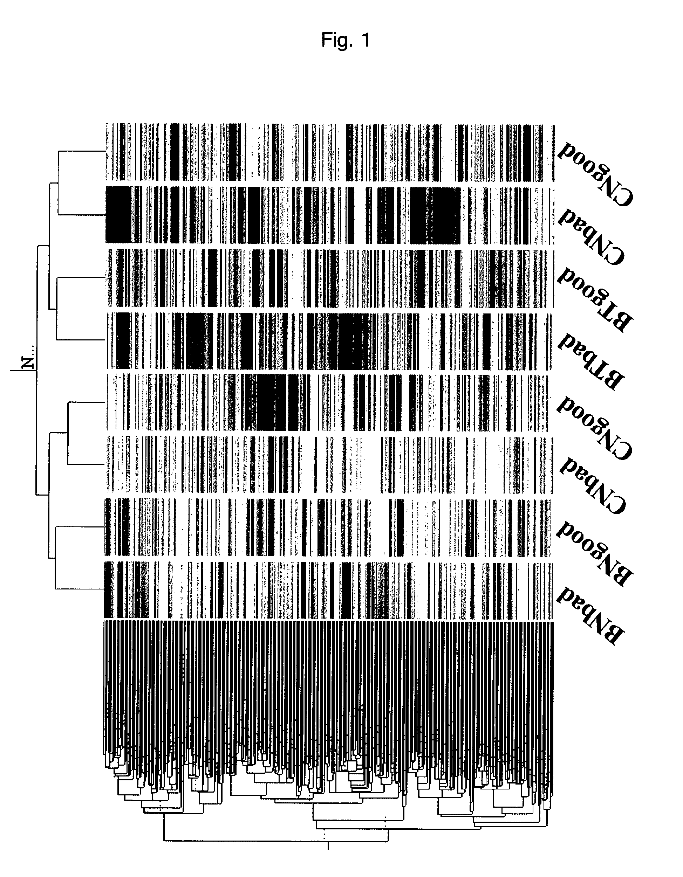

Figure 1 is a view showing the phylogenetic tree of samples obtained from the entire gene expression profile. Genes are rearranged based on the similarity in expression manner among samples, and further, samples are rearranged based on the similarity in the expression manner of the entire genes. Thus, the genetic affiliation is expressed in the form of a phylogenetic tree.

### BEST MODE FOR CARRYING OUT THE INVENTION

The present invention will be described in detail below.

The present invention is characterized in that the follow-up clinical data collected for a long period of time after the resection of hepatocellular carcinoma are divided into a poor prognosis case group (for example, a case group wherein the cancer recurs within 1 year, leading to death within 2 years) and into a good prognosis case group (for example, a case group wherein the cancer does not recur for 4 or more years), and is characterized in that a gene causing poor prognosis or a gene causing good prognosis (for example, a gene associated with promotion of the recurrence and a gene associated with suppression of the recurrence) is identified based on the characteristics of a gene group, which is expressed in the excised liver tissues. The present invention relates to classification of causal viruses into type B hepatocellular carcinoma cases and into type C hepatocellular carcinoma cases based on clinical data, and identification of a gene having a prognostic correlation from each of the tissues of a nontumor tissue and the tissues of a tumor tissue.

The gene of the present invention is obtained by analyzing the correlation between tissues actually collected from a patient and a pathologic condition thereof, and thereby clarifying the type of a case, a pathologic condition, and a gene, which are used to clarify the correlation between a gene and a pathologic condition.

### 1. Classification of test samples

The postoperative course is observed after an operation to resect liver cancer, and test samples are classified into an early recurrence group and into a late recurrence group.

The term "early recurrence group" is used to mean a case group wherein the cancer recurs within a certain period of time after resection, thereafter leading to death. A recurrence period is not particularly limited. For example, it is 1 year or shorter, or 2 years or shorter. A survival time is not particularly limited either. For example, it is 1 year or shorter, 2 years or shorter, or 3 years or shorter, after recurrence. The term "late recurrence group" is used to mean a case group wherein the cancer does not recur for a certain period of time after resection (for example, 3 years or longer, and preferably 4 years or longer).

In reality, 51 cases, which were subjected to an operation to resect hepatocellular carcinoma at stages I and II, were used as targets. The 51 cases contain 16 cases of type B hepatocellular carcinoma and 35 cases of type C hepatocellular carcinoma. Based on the follow-up clinical data of such cases, 2 cases were selected from the type B hepatocellular carcinoma and 3 cases were selected from the type C hepatocellular carcinoma, and these cases were classified into an early recurrence group. On the other hand, 2 cases selected from the type B hepatocellular carcinoma and 3 cases were selected from the type C hepatocellular carcinoma, and these cases were classified into a late recurrence group. With regard to the RNA portions of the nontumor tissues and tumor tissues of such 10 cases, the following expression profile analysis was carried out.

### 2. Gene analysis

Total RNA is extracted from each type of the liver tissues of the classified groups, and gene expression profiles are then compared between the groups using a microarray. Such total RNA can be extracted using a commercially available reagent (for example, TRIzol). For detection of an expression profile, Microarray (Affymetrix) is used, for example.

Moreover, the present invention enables the analysis of a gene, which changes expression in the tissues of a nontumor tissue as well as in the tissue of a tumor tissue. The term "nontumor tissue" is used herein to mean liver tissues involved in a resection of hepatocellular carcinoma, which do not contain cancer cells. However, such a "nontumor tissue" does not necessarily mean normal liver tissues, but it also includes tissues affected by chronic hepatitis (hepatitis B or hepatitis C) or liver cirrhosis. For example, a gene up-regulated in a nontumor tissue in a late recurrence group including type B hepatocellular carcinoma cases or type C hepatocellular carcinoma cases, wherein almost all tissues are such affected tissues, can be used as an analysis target. In the case of such tissues affected by chronic hepatitis or liver cirrhosis, a necrotic inflammatory reaction, regenerating nodules, fibrosis attended with decidual liver cells, or the like are observed. Among such cells, there are cells, which can be potential cells causing the development of hepatocellular carcinoma. Accordingly, it is considered that gene expression relevant to prognosis exists in the nontumor tissue. Thus, prognosis (for example, recurrence) can be predicted using such gene expression as an indicator (for example, by analyzing changes in such gene expression).

A gene used for evaluation of cancer is identified based on the correlation of changes in gene expression with phenotype (recurrence, early progression, etc.). The term "evaluation of cancer" is used to mean evaluation regarding the pathologic conditions of cancer or the stage of cancer progression. Such evaluation of cancer includes prediction of the presence or absence of metastasis or recurrence.

The present invention provides an up-regulated gene or a down-regulated gene in terms of recurrence. The term "recurrence" is used to mean that a lesion, which is considered to be a new carcinoma, appears in the liver, after a treatment for a primary lesion has been determined to complete.

### 3. Evaluation of gene

Using disease model cells or animals, the identified gene is evaluated in terms of availability as a factor of suppressing the development of pathologic conditions. Namely, (1) the remaining cases of hepatocellular carcinoma, the prognosis of which has been known, are subjected to quantitative analysis of gene expression, and the correlation with the prognosis is studied. (2) The gene is transferred into a hepatocellular carcinoma-cultured cell line, and it is allowed to express therein. Thereafter, the cell growth and a change in malignancy are evaluated based on ability to form colonies in a soft agar plate or ability to form tumors in nude mice. (3) Using a cultured hepatic cell line established from a patient with chronic hepatitis, the gene is transferred into the cells, and it is allowed to express therein. Thereafter, the cell growth and malignant transformation are evaluated by the same method as that described in (2) above. (4) The gene is transferred into the liver of a hepatocellular carcinoma development-model animal, and it is allowed to express therein. Thereafter, the course up to the development of liver cancer is evaluated.

In (1) above, the quantitative analysis of gene expression is carried out by real-time PCR, for example. That is to say, a commercially available reverse transcriptase is used for the total RNA as produced above, so as to synthesize cDNA. As a PCR reagent, a commercially available reagent can be used. Moreover, PCR may be carried out in accordance with commercially available protocols. For example, preliminary heating is carried out at 95°C for 10 minutes, and thereafter, a cycle consisting of 95°C for 15 seconds and 60°C (or 65°C) for 60 seconds, is repeated 40 times. Examples of an internal standard gene used herein as a target may include housekeeping genes such as glyceraldehyde 3-phosphatase dehydrogenase (GAPDH), 18S ribosomal RNA (18S rRNA), β-Actin, cyclophilin A, HPRTl (hypoxanthine phosphoribosyltransferase 1), B2M (beta-2 microglobulin), ribosomal protein L13a, or ribosomal protein L4. Persons skilled in the art can appropriately select such an internal standard gene. As an analysis method, absolute quantitative analysis or relative quantitative analysis of an expression level is adopted. The absolute quantitative analysis is preferable. Herein, absolute quantification of an expression level is obtained by determining a threshold line on which a calibration curve becomes optimum and then obtaining the number of threshold PCR cycles and a threshold cycle value (Ct) of each sample. On the other hand, a relative expression level is expressed with a Δ Ct value obtained by subtracting the Ct value of an internal standard gene (for example, GAPDH) from the Ct value of a target gene. Values obtained using the formula (2(^{-ΔCt})) can be used for evaluation of a linear expression level.

When a calibration curve is produced, values obtained by subjecting standard samples to serial dilution and simultaneous measurement (the samples are placed in a single plate and simultaneously measured, using a single reaction solution) may be used.

When an absolute expression level can be obtained relative to a calibration curve, the absolute expression level of a target gene and that of an internal standard gene are obtained, and the ratio of the target gene expression level/the internal standard gene expression level is calculated for each sample, so as to use it for evaluation.

Genes are selected from the results of the microarray of a late recurrence group and that of an early recurrence group. Thereafter, among genes, regarding which the results of real-time PCR obtained by the aforementioned method correspond with the results of the microarray, those exhibiting a correlation with a recurrence period can be identified as up-regulated genes of non tumor tissue, for example.

As described above, as genes identified as an up-regulated gene, various genes can be selected depending on experimental conditions applied during the identification, such as an internal standard gene, a primer sequence, or an annealing temperature which are used. Also, using various types of statistical methods (for example, Mann-Whitney U test), a gene correlating to a recurrence period can be selected.

The full-length sequence of the gene of the present invention can be obtained as follows. That is to say, it is searched through DNA database, and it can be obtained as known sequence information. Otherwise, the above full-length sequence is isolated from human liver cDNA library by hybridization screening.

In the present invention, genes up-regulated in cases where the cancer has not recurred at an early date (late recurrence) include those shown in Tables 1 to 4. On the other hand, genes up-regulated in cases where the cancer has recurred at an early date include those shown in Tables 5 to 8.
Table 1: Genes (24) up-regulated in a nontumor tissue in a late recurrence group of type B hepatocellular carcinoma cases
Table 2: Genes (10) up-regulated in a nontumor tissue in a late recurrence group of type C hepatocellular carcinoma cases
Table 3: Genes (137) up-regulated in a tumor tissue in a late recurrence group of type B hepatocellular carcinoma cases
Table 4: Genes (104) up-regulated in a tumor tissue in a late recurrence group of type C hepatocellular carcinoma cases
Table 5: Genes (48) up-regulated in a nontumor tissue in an early recurrence group of type B hepatocellular carcinoma cases
Table 6: Genes (12) up-regulated in a nontumor tissue in an early recurrence group of type C hepatocellular carcinoma cases
Table 7: Genes (75) up-regulated in a tumor tissue in an early recurrence group of type B hepatocellular carcinoma cases
Table 8: Genes (38) up-regulated in a tumor tissue in an early recurrence group of type C hepatocellular carcinoma cases

**Table 1 Genes (24) up-regulated in nontumor tissue in late recurrence group of hepatitis B cases (BNgood)**

| No. | Gene | Overlapped group |
|---|---|---|
| 1 | TNFSF14 | |
| 2 | MMP2 | |
| 3 | SAA2 | Late recurrence group (type B, tumor) |
| 4 | COL1A1 | |
| 5 | COL1A2 | |
| 6 | DPYSL3 | |
| 7 | PPARD | |
| 8 | LUM | |
| 9 | MSTP032 | |
| 10 | CRP | |
| 11 | TRIM38 | |
| 12 | S100A6 | |
| 13 | PZP | |
| 14 | EMP1 | |
| 15 | A1590053 | |
| 16 | MAP3K5 | |
| 17 | TIMP1 | |
| 18 | GSTM1 | Late recurrence group (type B, tumor) Late recurrence group (type C, tumor) |
| 19 | CSDA | |
| 20 | GSTM2 | Late recurrence group (type B, tumor) Late recurrence group (type C, tumor) |
| 21 | SGK | Late recurrence group (type B, tumor) |
| 22 | LMNA | |
| 23 | MGP | |
| 24 | LTBP2 | |

**Table 2 Genes (10) up-regulated in nontumor tissue in late recurrence group of hepatitis C cases (CNgood)**

| No. | Gene | Overlapped group |
|---|---|---|
| 25 | M10098 | Late recurrence group (type B, tumor) Late recurrence group (type C, tumor) |
| 26 | PSMB8 | |
| 27 | RALGDS | |
| 28 | APOL3 | |
| 29 | GBP1 | |
| 30 | RPS14 | |
| 31 | CXCL9 | |
| 32 | DKFZp564F212 | |
| 33 | CYP1B1 | |
| 34 | TNFSF10 | |

**Table 3 Genes (137) up-regulated in tumor tissue in late recurrence group of hepatitis B cases (BTgood)**

| No. | Gene | Overlapped group | |
|---|---|---|---|
| 35 | HP | | |
| 25 | M10098 | | Late recurrence group (type C. tumor) Late recurrence group (type C. nontumor) |
| 36 | CYP2E1 | | |
| 37 | HDL | | Late recurrence group (type C. tumor) |
| 38 | GPX4 | | |
| 39 | GOS2 | | |
| 40 | HA02 | | |
| 41 | ATF5 | | Late recurrence group (type C. tumor) |
| 42 | MTIF | | Late recurrence group (type C. tumor) |
| 43 | CYP3A4 | | Late recurrence group (type C. tumor) |
| 44 | Scd | | |
| 45 | SERPINA7 | | |
| 46 | AKR1D1 | | |
| 47 | AL031602 | | |
| 48 | TSC501 | | |
| 18 | GSTM1 | Late recurrence group (type B. nontumor) | Late recurrence group (type C. tumor) |
| 3 | SAA2 | Late recurrence group (type B, nontumor) | |
| 49 | BHMT | | Late recurrence group (type C. tumor) |
| 50 | HADHSC | | |
| 51 | FBXO9 | | |
| 52 | KIAA0442 | | |
| 53 | KIAA0293 | | Late recurrence group (type C. tumor) |
| 54 | IGHG3 | | |
| 55 | ADH2 | | Late recurrence group (type C. tumor) |
| 20 | GSTM2 | Late recurrence group (type B. nontumor) | Late recurrence group (type C. tumor) |
| 56 | PPIF | | |
| 57 | ALDH8A1 | | |
| 58 | IGLJ3 | | |
| 59 | HCN3 | | |
| 60 | ADH6 | | Late recurrence group (type C. tumor) |
| 61 | AK02720 | | Late recurrence group (type C. tumor) |
| 62 | NET-6 | | |
| 63 | CYP2D6 | | |
| 64 | MAFB | | |
| 65 | GHR | | |
| 66 | KHK | | |
| 67 | ADFP | | |
| 68 | LCE | | |
| 69 | MPDZ | | Late recurrence group (type C. tumor) |
| 70 | TEM6 | | |
| 71 | KIAA0914 | | |
| 72 | KLKB1 | | |
| 73 | M11167 | | Late recurrence group (type C. tumor) |
| 21 | SGK | Late recurrence group (type B. nontumor) | |
| 74 | EHHADH | | |
| 75 | MBL2 | | Late recurrence group (type C. tumor) |
| 76 | APP | | |
| 77 | MT1G | | |
| 78 | TPD52L1 | | Late recurrence group (type C, tumor) |
| 79 | CXCL10 | | |
| 80 | AI972416 | | |
| 81 | FCGR2B | | |
| 82 | IGL@ | | |
| 83 | FLJ10134 | | |
| 84 | PPAP2B | | |
| 85 | CDC42 | | |
| 86 | HBA2 | | |
| 87 | CYP1A2 | | Late recurrence group (type C. tumor) |
| 88 | CYP2B6 | | |
| 89 | DKFZP586B1621 | | |
| 90 | MTP | | |
| 91 | X07868 | | |
| 92 | RNAHP | | Late recurrence group (type C. tumor) |
| 93 | HLF | | Late recurrence group (type C. tumor) |
| 94 | PPP1R3C | | |
| 95 | CDC2L2 | | |
| 96 | NRIP1 | | |
| 97 | GPD1 | | |
| 98 | KIAA1053 | | |
| 99 | CCL19 | | |
| 100 | CRI1 | | |
| 101 | THBS1 | | Late recurrence group (type C. tumor) |
| 102 | SLC5A3 | | |
| 103 | GADD45B | | |
| 104 | AGL | | |
| 105 | ADK | | |
| 106 | IGKC | | |
| 101 | CYP2A6 | | Late recurrence group (type C. tumor) |
| 108 | GADD45A | | Late recurrence group (type C. tumor) |
| 109 | FLJ20701 | | |
| 110 | LOC57826 | | |
| 111 | SLC2A2 | | |
| 112 | CIRBP | | |
| 113 | CGI-26 | | |
| 114 | DEFB1 | | |
| 115 | HMGCS1 | | |
| 116 | ODC1 | | |
| 117 | GLUL | Early recurrence group (type B. nontumor) | Late recurrence group (type C. tumor) |
| 118 | CYP27A1 | | |
| 119 | SULT2A1 | | Late recurrence group (type C. tumor) |
| 120 | AK024828 | | |
| 121 | PHLDA1 | | |
| 122 | NR112 | | |
| 123 | MSRA | | |
| 124 | RNASE4 | | |
| 125 | AI339732 | | |
| 126 | HBA2 | | |
| 127 | AL050025 | | |
| 128 | CSAD | | |
| 129 | SID6-306 | | |
| 130 | NM024561 | | |
| 131 | BCKDK | | |
| 132 | SLC6A1 | | |
| 133 | CG018 | | |
| 134 | GNE | | |
| 135 | CKLFSF6 | | |
| 136 | COMT | | |
| 137 | AL135960 | | |
| 138 | KIAA0179 | | |
| 139 | c-maf | | |
| 140 | OSBPL11 | | |
| 141 | R06655 | | Late recurrence group (type C. tumor) |
| 142 | KIAA04461 | | |
| 143 | IGF1 | | Late recurrence group (type C. tumor) |
| 144 | HBA1 | | Late recurrence group (type C,tumor) |
| 145 | LOC55908 | | |
| 146 | ENPEP | | |
| 147 | TXNIP | | |
| 148 | KIAA0624 | | |
| 149 | ENPP1 | | |
| 150 | CYP4F3 | | |
| 151 | CAV2 | | |
| 152 | BE908931 | | |
| 153 | LECT2 | | |
| 154 | MLLT2 | | |
| 155 | FLR1 | | |
| 156 | TF | | |
| 157 | DAO | | |
| 158 | A1620911 | | |
| 159 | GBP1 | | |
| 160 | UGP2 | | |
| 161 | GADD45B | | |
| 162 | SC4MOL | | |
| 163 | BE908931 | | |
| 164 | TUBB | | |
| 165 | EPHX2 | | |
| 166 | SORD | | |

**Table 4 Genes (104) up-regulated in tumor tissue in late recurrence group of hepatitis C cases (CTgood)**

| No. | Gene | Overlapped group | | |
|---|---|---|---|---|
| 167 | LEAP-1 | | | |
| 168 | PPD | | | |
| 37 | HDL | | Late recurrence group (type B. tumor) | |
| 43 | CYP3A4 | | Late recurrence group (type B. tumor) | |
| 107 | CYP2A6 | | Late recurrence group (type B, tumor) | |
| 25 | M10098 | Late recurrence group (type C. nontumor) | Late recurrence group (type B. tumor) | |
| 169 | RACE | | | |
| 170 | SLC27A5 | | | |
| 171 | FLJ20581 | | | |
| 172 | FLJ10851 | | | |
| 53 | KlAA0293 | | Late recurrence group (type B. tumor) | |
| 173 | C9 | | | |
| 174 | AL354872 | | | |
| 175 | AKR1C1 | | | |
| 176 | PCK1 | | | |
| 18 | GSTM1 | | Late recurrence group (type B. tumor) | Late recurrence group (type B. nontumor) |
| 87 | CYP1A2 | | Late recurrence group (type B. tumor) | |
| 177 | ANGPTL4 | | | |
| 178 | AOX1 | | | |
| 179 | SDS | | | |
| 20 | GSTM2 | | Late recurrence group (type B. tumor) | Late recurrence group (type B, nontumor) |
| 73 | M11167 | | Late recurrence group (type B. tumor) | |
| 180 | CYP2C9 | | | |
| 181 | SIPL | | | |
| 182 | GLYAT | | | |
| 75 | MBL2 | | Late recurrence group (type B. tumor) | |
| 183 | CYP1A1 | | | |
| 184 | CRP | | | |
| 141 | R06655 | | Late recurrence group (type B. tumor) | |
| 185 | ACADL | | | |
| 93 | HLF | | Late recurrence group (type B. tumor) | |
| 186 | NR113 | | | |
| 187 | CA2 | | | |
| 188 | CYP2C8 | | | |
| 189 | PON1 | | | |
| 55 | ADH2 | | Late recurrence group (type B, tumor) | |
| 92 | RNAHP | | Late recurrence group (type B. tumor) | |
| 190 | AQP9 | | | |
| 119 | SULT2A1 | | Late recurrence group (type B. tumor) | |
| 191 | SPP1 | | | |
| 192 | K]AA0934 | | | |
| 193 | AKAP12 | | | |
| 194 | APOF | | | |
| 195 | FM03 | | | |
| 196 | SLC22A1 | | | |
| 197 | DCXR | | | |
| 198 | CYP3A7 | | | |
| 199 | SOCS2 | | | |
| 101 | THBS1 | | Late recurrence group (type B. tumor) | |
| 41 | ATF5 | | Late recurrence group (type B. tumor) | |
| 200 | BCRP | | | |
| 60 | ADH6 | | Late recurrence group (type B. tumor) | |
| 201 | humNRDR | | | |
| 202 | GADD45G | | | |
| 203 | SRD5A1 | | | |
| 204 | ABCA8 | | | |
| 61 | AK026720 | | Late recurrence group (type B, tumor) | |
| 205 | APOC4 | | | |
| 206 | FTHFD | | | |
| 207 | ISG15 | | | |
| 208 | IGFBP2 | | | |
| 49 | BHMT | | Late recurrence group (type B. tumor) | |
| 209 | DNASE1L3 | | | |
| 210 | SRD5A1 | | | |
| 211 | E21G4 | | | |
| 212 | COL1A2 | | | |
| 213 | C20orf46 | | | |
| 214 | ESR1 | | | |
| 215 | BLVRB | | | |
| 216 | LRP16 | | | |
| 217 | SLC1A1 | | | |
| 218 | ABCB6 | | | |
| 69 | MPDZ | | Late recurrence group (type B. tumor) | |
| 219 | FBP1 | | | |
| 220 | ALAS1 | | | |
| 221 | JFIT1 | | | |
| 222 | PPARGC1 | | | |
| 223 | Id-1H | | | |
| 224 | RBP1 | | | |
| 225 | CSHMT | | | |
| 226 | LOC155066 | | | |
| 42 | MT1F | | Late recurrence group (type B. tumor) | |
| 227 | AGXT2L1 | | | |
| 228 | TIMM17A | | | |
| 229 | SEC14L2 | | | |
| 230 | MAOA | | | |
| 231 | MYC | | | |
| 232 | ACAA2 | | | |
| 233 | AL109671 | | | |
| 234 | ABCA6 | | | |
| 143 | IGF1 | | Late recurrence group (type B. tumor) | |
| 235 | GRHPR | | | |
| 236 | HADH2 | | | |
| 237 | AFM | | | |
| 238 | COL1A1 | | | |
| 239 | MTHFD1 | | | |
| 240 | NMT2 | | | |
| 108 | GADD45A | | Late recurrence group (type B. tumor) | |
| 241 | UGT2B15 | | | |
| 242 | AR | | | |
| 78 | TPD52L1 | | Late recurrence group (type B. tumor) | |
| 243 | sMAP | | | |
| 117 | GLUL | Early recurrence group (type B. nontumor) | Late recurrence group (type B. tumor) | |
| 244 | dJ657E11.4 | | | |

**Table 5 Genes (48) up-regulated in nontumor tissue in early recurrence group of hepatitis B cases (BNbad)**

| No. | Gene | Overlapped group | |
|---|---|---|---|
| 245 | CTH | | Early recurrence group (type B, tumor) |
| 246 | OAT | | |
| 247 | PRODH | | Early recurrence group (type B, tumor) |
| 248 | CYP3A7 | | |
| 249 | DDT | | Early recurrence group (type B, tumor) |
| 250 | PGRMC1 | | |
| 251 | AKR1C1 | | |
| 252 | HGD | | Early recurrence group (type B, tumor) |
| 253 | FHR-4 | | |
| 254 | AL354872 | | |
| 255 | FST | | Early recurrence group (type B, tumor) |
| 256 | COX4 | | |
| 257 | APP | | |
| 258 | PSPHL | | |
| 259 | CYP1A1 | | |
| 260 | ZNF216 | | |
| 261 | LEPR | | Early recurrence group (type B, tumor) |
| 262 | TOM1L1 | | |
| 263 | PECR | | |
| 264 | ALDH7A1 | | |
| 265 | GNMT | | |
| 266 | OATP-C | | |
| 267 | AKR1B10 | Early recurrence group (type C, nontumor) | Early recurrence group (type B, tumor) |
| 268 | ANGPTL3 | | |
| 269 | AASS | | |
| 270 | CALR | | |
| 271 | BAAT | | |
| 272 | PMM1 | | |
| 273 | RAB-R | | |
| 117 | GLUL | Late recurrence group (type C, tumor) | Late recurrence group (type B, tumor) |
| 274 | CSHMT | | |
| 275 | UGT1A3 | | |
| 276 | HSPG1 | | |
| 277 | QPRT | Early recurrence group (type C, nontumor) | |
| 278 | DEPP | | |
| 279 | CA2 | | Early recurrence group (type B, tumor) |
| 280 | FTHFD | | |
| 281 | LAMP1 | | |
| 282 | FKBP1A | | |
| 283 | BNIP3 | | |
| 284 | MAP3K12 | | |
| 285 | ASS | | Early recurrence group (type B, tumor) |
| 286 | ACTB | | |
| 287 | PLAB | | Early recurrence group (type B, tumor) |
| 288 | ENO1L1 | | |
| 289 | IGFBP3 | | |
| 290 | UK114 | | |
| 291 | ERF-1 | | |

**Table 6 Genes (12) up-regulated in nontumor tissue in early recurrence group of hepatitis C cases (CNbad)**

| No. | Gene | Overlapped group | |
|---|---|---|---|
| 292 | ALB | | |
| 293 | NR0B2 | | |
| 267 | AKR1B10 | Early recurrence group (type B, nontumor) | Early recurrence group (type B, tumor) |
| 294 | MAFB | | |
| 295 | BF530535 | | |
| 296 | MRPL24 | | |
| 297 | DSIPI | | |
| 277 | QPRT | Early recurrence group (type B. nontumor) | |
| 298 | VNN1 | | |
| 299 | IRS2 | | |
| 300 | FMO5 | | |
| 301 | DCN | | |

**Table 7 Genes (75) up-regulated in tumor tissue in early recurrence group of hepatitis B cases (BTbad)**

| No. | Gene | Overlapped group | | |
|---|---|---|---|---|
| 247 | PRODH | Early recurrence group (type B, nontumor) | | |
| 302 | PLA2G2A | | Early recurrence group (type C. tumor) | |
| 303 | SDS | | | |
| 304 | LGALS3BP | | | |
| 305 | BACE2 | | | |
| 261 | LEPR | Early recurrence group (type B. nontumor) | | |
| 306 | RCN1 | | | |
| 307 | MRC1 | | | |
| 308 | TM4SF5 | | | |
| 309 | NK4 | | | |
| 310 | PABL | | | |
| 311 | IGFBP2 | | | |
| 312 | GRINA | | | |
| 313 | IFI27 | | | |
| 314 | GP2 | | | |
| 315 | GA | | | |
| 316 | P4HA2 | | | |
| 317 | KYNU | | | |
| 318 | PCK1 | | | |
| 319 | UQBP | | | |
| 320 | HLA-DRB1 | | | |
| 252 | HGO | Early recurrence group (type B. nontumor) | | |
| 321 | HTATIP2 | | | |
| 322 | GGT1 | | | |
| 323 | CTSH | | | |
| 324 | MVP | | | |
| 325 | SLC22A1L | | | |
| 326 | GMNN | | | |
| 327 | COM1 | | | |
| 328 | TM7SF2 | | | |
| 245 | CTH | Early recurrence group (type B. nontumor) | | |
| 329 | KDELR3 | | | |
| 330 | VPS28 | | | |
| 279 | CA2 | Early recurrence group (type B. nontumor) | | |
| 331 | SFN | | | |
| 332 | NM023948 | | | |
| 333 | OPLAH | | | |
| 334 | DGCR6 | | | |
| 335 | INSIG1 | | | |
| 267 | AKR1B10 | Early recurrence group (type B. nontumor) | | Early recurrence group (type C, nontumor) |
| 336 | PTGDS | | Early recurrence group (type C, tumor) | |
| 337 | SLC25A15 | | | |
| 338 | SEPW1 | | | |
| 339 | CD9 | | | |
| 340 | UOCRB | | | |
| 285 | ASS | Early recurrence group (type B. nontumor) | | |
| 341 | CPT1A | | | |
| 287 | PLAB | Early recurrence group (type B, nontumor) | | |
| 342 | GPAA1 | | | |
| 343 | HF1 | | | |
| 344 | GPX2 | | | |
| 345 | COPEB | | | |
| 346 | NDRG1 | | | |
| 347 | SYNGR2 | | | |
| 348 | GOT1 | | | |
| 349 | POLR2K | | | |
| 350 | AATF | | | |
| 255 | FST | Early recurrence group (type B. nontumor) | | |
| 351 | OAZIN | | | |
| 352 | RPL7 | | | |
| 353 | KIAA0128 | | | |
| 354 | CLDN7 | | | |
| 355 | ABCB6 | | | |
| 356 | GK | | | |
| 357 | LU | | Early recurrence group (type C, tumor) | |
| 358 | TNFSF4 | | | |
| 359 | OSBPL9 | | | |
| 360 | GSN | | | |
| 361 | LGALS4 | | | |
| 249 | DDr | Early recurrence group (type B. nontumor) | | |
| 362 | EIF3S3 | | | |
| 363 | SLC12A2 | | | |
| 364 | RAMP1 | | | |
| 365 | HSPB1 | | | |
| 366 | Al201594 | | | |

**Table 8 Genes (38) up-regulated in tumor tissue in early recurrence group of hepatitis C cases (CTbad)**

| No. | Gene | Overlapped group |
|---|---|---|
| 367 | BL34 | |
| 368 | AL022324 | |
| 369 | IGHM | |
| 370 | TXNIP | |
| 371 | FSTL3 | |
| 372 | AW978896 | |
| 373 | NM018687 | |
| 374 | L48784 | |
| 375 | AJ275355 | |
| 376 | PER1 | |
| 377 | CYBA | |
| 302 | PLA2G2A | Early recurrence group (type B, tumor) |
| 378 | SGK | |
| 379 | FKBP11 | |
| 380 | AI912086 | |
| 381 | IGLJ3 | |
| 382 | IGKC | |
| 336 | PTGDS | Early recurrence group (type B, tumor) |
| 383 | M20812 | |
| 384 | AGRN | |
| 385 | IL2RG | |
| 386 | X07868 | |
| 387 | PKM2 | |
| 388 | FGFR3 | |
| 389 | TRB@ | |
| 390 | TNFAIP3 | |
| 391 | TTC3 | |
| 392 | LPA | |
| 393 | AL049987 | |
| 394 | IER5 | |
| 395 | BSG | |
| 396 | TM4SF3 | |
| 397 | HMGB2 | |
| 357 | LU | Early recurrence group (type B, tumor) |
| 398 | CCL19 | |
| 399 | PAM | |
| 400 | PIK3R1 | |
| 401 | RANGAP1 | |

In Table 5, "CTH'' and "AL354872'' are genes, which encode the same protein.

The above-described genes can be included in a kit for evaluating cancer, singly or in combination, as appropriate. Examples of a gene set consisting of several genes may include those shown in Table 16 (described later). The above genes may have the partial sequence thereof. Such genes can be used as probes for detecting the expression of the genes shown in the table.

Moreover, the kit of the present invention may comprise primers used for gene amplification, a buffer solution, polymerase, etc.

With regard to such primers used for gene amplification, the DNA sequence and mRNA sequence of each gene sequence are obtained from database, and in particular, information including the presence or absence of a variant and exon-intron structure is obtained. The same sequences as sequences of portions corresponding to coding regions are used as target. One primer is intended to bridge over an adjacent exon, and it is designed such that only mRNA is detected. Otherwise, primer candidates are obtained using the web software "Primer3" (provided by Steve Rozen and Whitehead Institute for Biomedical Research), and thereafter, homology search is carried out using BLAST (NCBI) search, so as to select primers, which are able to avoid miss-annealing to similar sequences.

The sequence numbers of preferred primers are represented by the general formulas 2n-1 and 2n (wherein n represents an integer between 1 and 114). In the present invention, a primer represented by 2n-1 and a primer represented by 2n can be used as a set of primers. For example, when n is 1, a primer set consisting of the primers shown in SEQ ID NOS: 1 and 2 can be used, and when n is 2, a primer set consisting of the primers shown in SEQ ID NOS: 3 and 4 can be used. Particularly preferred primers can be obtained, when n is 2, 4, 7, 9, or 17.

Moreover, in (1) above, it is also possible to carry out the quantitative analysis of gene expression via immuno-dot blot assay or immunostaining. Such immuno-dot blot assay or immunostaining can be carried out according to common methods using an antibody reacting with the expression products of the genes shown in Tables 1 to 8. As such an antibody, a commercially available antibody may be used, or an antibody obtained by immunization of animals such as a mouse, a rat, or a rabbit, may also be used.

The present invention will be more specifically described in the following examples. However, these examples are not intended to limit the technical scope of the present invention.

### Example 1

Detection of up-regulated gene in hepatocellular carcinoma cases

As described below, using human hepatic tissues obtained from type B and type C hepatocellular carcinoma cases, molecules for suppressing the recurrence of hepatocellular carcinoma were identified at a gene level.

In order to understand a recurrence mechanism occurring after an operation to resect hepatocellular carcinoma and determine a gene capable of predicting the presence or absence of recurrence, gene expression profile analysis was carried out, using several cases, the recurrence periods of which were different. 51 cases, which were at stages I and II based on TNM classification, were used as targets. 5 cases wherein the cancer had not recurred for 4 or more years after the operation, and 5 cases wherein the cancer had recurred within 1 year after the operation, were selected. Thereafter, expression analysis was carried out using an HG-U133A array manufactured by Affymetrix.

The TRIzol reagent (Life Technologies, Gaithersburg, MD) was added to frozen tissues, and the obtained mixture was then homogenated with Polytron. Thereafter, chloroform was added to the homogenate, and they were then fully mixed, followed by centrifugation. After completion of the centrifugation, the supernatant was recovered, and an equivalent amount of isopropanol was added thereto. Thereafter, the precipitate of total RNA was recovered by centrifugation.

Type B hepatocellular carcinoma cases (wherein the causal virus is a hepatitis B virus) were divided into the following groups: the nontumor tissues and tumor tissues of 2 early recurrence cases; and the nontumor tissues and tumor tissues of 2 late recurrence cases. Also, type C hepatocellular carcinoma cases (wherein the causal virus is a hepatitis C virus) were divided into the following groups: the nontumor tissues and tumor tissues of 3 early recurrence cases; and the nontumor tissues and tumor tissues of 3 late recurrence cases. Thus, the total 8 groups were subjected to expression analysis.

For each sample group, 15 µg of total RNA was prepared. Thereafter, biotin-labeled cRNA was synthesized based on GeneChip Expression Analysis Technical Manual by Affymetrix. Using T7-(dt)₂₄ primer and Superscript II reverse transcriptase (Invitrogen Life Technology), the reaction was carried out for 1 hour, so as to synthesize first strand cDNA. Thereafter, *E. coli* DNA ligase, *E. coli* DNA polymerase, and *E. coli* RNase H were added thereto, and the obtained mixture was then allowed to react at 16°C for 2 hours. Finally, T4 DNA polymerase was added to the reaction product, so as to synthesize double strand cDNA. After cleanup of the cDNA, the BioArray high yield RNA transcript labeling kit (Affymetrix, Inc, CA) was used for *in vitro* transcription at 37°C for 4 hours, so as to synthesize biotin-labeled cRNA. A hybridization probe solution was prepared based on the Technical Manual, and the above solution was then added to GeneChip HG-U133A (Affymetrix, Inc, CA; containing 22,283 human genes), obtained by pre-hybridization at 45°C for 45 minutes. Thereafter, hybridization was carried out at 45°C for 16 hours. Thereafter, the reaction product was washed with GeneChip Fluidics Station 400 (Affymetrix, Inc, CA), and was then stained with streptavidin phycoerythrin and biotinylated antistreptavidin. Thereafter, the resultant was subjected to scanning using an HP GeneArray scanner (Affymetrix, Inc, CA).

The obtained data was analyzed using GeneSpring ver.5.0 (SiliconGenetics, Redwood, CA). After completion of normalization, using the signal of the control gene BioB used for intrinsic quantification as a detection limit (corresponding to several copies per cell). A gene, which has a signal intensity of 100 or greater and also has a present flag in at least one chip, was defined as a target of the analysis. As a result, 7,444 genes were determined to be such analysis targets. In nontumor tissues, genes having 2.5 times or more difference between the early recurrence group and the late recurrence group have been identified. In tumor tissues, genes having 3 times or more difference between such two groups have been identified.

As a result, among the selected 7,444 genes, genes having 2.5 times or more difference between the absence and the presence of recurrence in nontumor tissues consisted of 34 up-regulated genes and 58 down-regulated genes. On the other hand, genes having 3 time or more difference between such two groups in tumor tissues consisted of 215 up-regulated genes and 110 down-regulated genes. Among these genes, as a gene up-regulated in the recurrence-absent group in both cases of type B and type C, no such genes were found in nontumor tissues, whereas 26 genes were found in tumor tissues. On the other hand, among these genes, as a gene up-regulated in the recurrence-present group in both cases of type B and type C, 2 genes were found in nontumor tissues, whereas 3 genes were found in tumor tissues. Moreover, there were genes up-regulated in both tumor and nontumor tissue. There were found 5 genes up-tegulated in the recurrence-absent group, and 10 genes up-regulated in the recurrence-present group (Table 9).

It is to be noted that the total is not 402 but 401 in Table 9. This is because the overlapping of GLUL is a particular case.

**Table 9 Genes associated with recurrence of hepatocellular carcinoma**

| | Up-regulated in late recurrence group | | Up-regulated in early recurrence group | | Both cases | |
|---|---|---|---|---|---|---|
| | nontumor tissue | tumor tissue | nontumor tissue | tumor tissue | | |
| Hepatitis B | 24 | 137 | | | 4 | |
| | | | 48 | 75 | | 10 |
| Hepatitis C | 10 | 104 | | | 1 | |
| | | | 12 | 38 | | 0 |
| Both types | 0 | 26 | | | | |
| | | | 2 | 3 | | |
| Total | 34 | 215 | | | 244 | |
| | | | 58 | 110 | | 158 |
| | | | | | Total | 401 |

From the results shown in Table 9, it can be said that with regard to a difference in recurrence prognosis, a change in gene expression is greater in a tumor tissue than in a nontumor tissue, and that such a change in gene expression is greater in type B hepatocellular carcinoma cases than in type C hepatocellular carcinoma cases. In addition, there are genes associated with recurrence prognosis, which are found independently of a causal virus, but unexpectedly, such genes are rare. As in the case of the development of cancer, it is considered that different mechanisms are involved in the recurrence of cancer, depending on the type of a causal virus.

In the analysis of a sample phylogenetic tree, the expression profiles of all genes are first divided into nontumor tissues and tumor tissues. In each of such nontumor tissues and tumor tissues, a genetic affiliation, which is not caused by recurrence prognosis but caused by a causal virus, was observed (Figure 1). In Figure 1, with regard to notation indicating each test group, such as "BNbad'' or "BNgood," the first alphabet indicates the type of a virus. That is, "B" represents hepatitis B virus, and "C" represents hepatitis C virus. The second alphabet "N" represents a nontumor tissue, and "T" represents a tumor tissue. Moreover, "bad" represents early recurrence, and "good" represents late recurrence.

It is considered that gene expression affecting recurrence prognosis is caused by a change in the gene expression of limited genes.

As stated above, candidate genes capable of clarifying a recurrence mechanism or predicting the presence or absence of recurrence were found (Tables 1 to 8).

### Example 2

Study of correlation between the recurrence period and an expression level of genes in each group in type C hepatocellular carcinoma cases

As mentioned below, with regard to genes up-regulated in the nontumor tissues of a late recurrence group and an early recurrence group in type C hepatocellular carcinoma cases, the correlation between the recurrence period and an expression level was studied.

The total 22 nontumor tissue samples, including 6 cases of type C hepatocellular carcinoma used in the gene expression profile analysis, were used as targets. The clinicopathological findings of each case and the recurrence period (that is, the period of time in which the cancer has not yet recurred) are shown in Table 10A.

**Table 10A Type C hepatocellular carcinoma cases**

| Case No. | Sex | Age | Nontumor tissue | stage | Number of months without recurrence | Microarray |
|---|---|---|---|---|---|---|
| 59 | M | 66 | CH | I | 84 | Late recurrence group |
| 18 | M | 68 | LC | I | 58 | Late recurrence group |
| 6 | M | 65 | CH | II | 51 | Late recurrence group |
| 25 | M | 51 | CH | I | 45 | |
| 29 | M | 70 | CH | II | 43 | |
| 12 | M | 66 | CH | II | 41 | |
| 4 | M | 65 | CH | I | 40 | |
| 48 | F | 65 | LC | I | 39 | |
| 31 | M | 60 | LC | I or II | 38 | |
| 16 | M | 70 | CH | I | 37 | |
| 22 | M | 65 | CH | I | 34 | |
| 3 | F | 71 | LC | I | 29 | |
| 65 | M | 60 | LC | I | 29 | |
| 30 | F | 62 | LC | II | 28 | |
| 10 | M | 56 | LC | I | 26 | |
| 23 | M | 62 | CH | II | 16 | |
| 26 | M | 70 | LC | I | 16 | |
| 14 | M | 62 | CH | II | 14 | Early recurrence group |
| 62 | M | 66 | LC | I | 13 | |
| 17 | M | 54 | LC | I | 12 | |
| 15 | F | 68 | LC | II | 8 | Early recurrence group |
| 44 | M | 58 | CH | I | 4 | Early recurrence group |

| | | | | | | |
|---|---|---|---|---|---|---|
| CH: chronic hepatitis; LC: liver cirrhosis | | | | | | |
| Stage of case 31: undetermined | | | | | | |
| The term "number of months without recurrence" includes not only the number of months required for recurrence, but also includes the investigation period in which recurrence was not observed. | | | | | | |

In addition, the cases shown in Table 10A were changed or revised as a result of follow-up study. Moreover, with regard to the total 35 cases, including cases added as the targets of the present example, the clinicopathological findings of each case and the recurrence period (that is, the period of time in which the cancer has not yet recurred) are shown in Table 10B.

**Table 10B Type C hepatocellular carcinoma cases**

| Case No. | Sex | Age | Nontumor tissue | stage | Number of months without recurrence | Microarray |
|---|---|---|---|---|---|---|
| 59 | M | 66 | CH | I | >94 | Late recurrence group |
| 6 | M | 65 | CH | II | 65 | Late recurrence group |
| 25 | M | 51 | CH | I | > 58 | |
| 18 | M | 68 | LC | I | 58 | Late recurrence group |
| 12 | M | 66 | CH | II | 41 | |
| 4 | M | 65 | CH | I | >40 | |
| 29 | M | 70 | CH | II | 39 | |
| 16 | M | 70 | CH | I | >37 | |
| 48 | F | 65 | LC | I | 37 | |
| 31 | M | 60 | LC | I | 37 | |
| 80 | M | 73 | CH | II | 34 | |
| 22 | M | 65 | CH | I | 33 | |
| 3 | F | 71 | LC | I | 29 | |
| 65 | M | 60 | LC | I | 28 | |
| 30 | F | 62 | LC | II | 26 | |
| 10 | M | 56 | LC | I | 25 | |
| 70 | M | 57 | LC | II | 24 | |
| 79 | M | 73 | LC | I | 22 | |
| 73 | M | 50 | CH | II | 20 | |
| 81 | F | 69 | LC | I | 17 | |
| 26 | M | 70 | LC | I | 16 | |
| 72 | M | 71 | LC | II | 16 | |
| 69 | M | 66 | LC | II | 15 | |
| 14 | M | 62 | CH | II | 14 | Early recurrence group |
| 78 | F | 66 | CH | I | 13 | |
| 82 | M | 71 | CH | I | 13 | |
| 17 | M | 54 | LC | I | 12 | |
| 71 | M | 57 | LC | II | 12 | |
| 77 | F | 65 | LC | I | 10 | |
| 62 | M | 66 | LC | I | 9 | |
| 74 | M | 67 | CH | II | 9 | |
| 15 | F | 68 | LC | II | 8 | Early recurrence group |
| 76 | M | 72 | NL | I | 7 | |
| 75 | M | 65 | CH | II | 6 | |
| 44 | M | 58 | CH | I | 4 | Early recurrence group |

| | | | | | | |
|---|---|---|---|---|---|---|
| CH: chronic hepatitis; LC: liver cirrhosis; NL: normal liver | | | | | | |
| The term "number of months without recurrence" includes not only the number of months required for recurrence, but also includes the period in which recurrence has not yet been observed at the time of investigation. | | | | | | |

With regard to the total 21 genes consisting of 9 genes (CNgood) up-regulated in the nontumor tissues of the late recurrence group shown in Table 2 and 12 genes (CNbad) up-regulated in the nontumor tissues of the early recurrence group shown in Table 6, the relationship between the recurrence period and an expression level was analyzed.

First, total RNA was extracted from the nontumor liver tissue of each case by the same method as that described in Example 1 above.

In order to eliminate the influence of DNA mixed therein, the total RNA was treated with DNase I (DNase I, TAKARA SHUZO, Kyoto, Japan) at 37°C for 20 minutes, and it was then purified again with a TRIzol reagent. Using 10 µg of the total RNA, a reverse transcription reaction was carried out with 100 µl of a reaction solution comprising 25 units of AMV reverse transcriptase XL (TAKARA) and 250 pmol of a 9-mer random primer.

Real-time PCR was carried out using 0.25 to 50 ng each of synthetic cDNA. 25 µl of a reaction solution, SYBR Green PCR Master mix (Applied Biosystems, Foster City, CA) was used, and ABI PRISM 7000 (Applied Biosystems) was employed. PCR was carried out under conditions wherein preliminary heating was carried out at 95°C for 10 minutes, and thereafter, a cycle consisting of 95°C for 15 seconds and 60°C (or 65°C) for 60 seconds, was repeated 40 to 45 times.

Using glyceraldehyde 3-phosphatase dehydrogenase (GAPDH) or 18S rRNA as an internal standard gene of each sample, relative quantitative analysis, and partially, absolute quantitative analysis, were carried out. Values obtained by subjecting standard samples to serial dilution and simultaneous measurement, were used to produce a calibration curve. A threshold line for optimization of such a calibration curve was determined, and the number of threshold PCR cycles, a threshold cycle value (Ct) was then obtained for each sample. A Δ Ct value was obtained by subtracting the Ct value of GAPDH or 18S rRNA from the Ct value of a target gene, and the obtained value was defined as the relative expression level of the target gene. Moreover, values obtained using the formula (2(^{-ΔCt})) were used for evaluation of a linear expression level.

On the other hand, with regard to genes whose absolute expression level can be calculated relative to a calibration curve, the absolute expression level of a target gene and that of an internal standard gene were obtained. Thereafter, the ratio of the target gene expression level/the internal standard gene expression level was calculated for each sample, and it was used for evaluation. All such measurements were carried out in a duplicate manner.

In Tables 11A, 11B, 12A, and 12B, the term "correspondence with microarray" is used to mean that when the ratio between the late recurrence group (case Nos. 59, 18, and 6) and the early recurrence group (case Nos. 14, 15, and 44) was obtained from the results of quantitative PCR performed on 6 cases (case Nos. 59, 18, 6, 14, 15, and 44 in Table 10A or 10B) used in the microarray analysis, genes, the above ratio of which was 1.5 or greater, corresponded with the results of the microarray in Example 1. Genes corresponding with the microarray results were indicated with the mark O. The above ratio is 1.5 or greater, and preferably 2 or greater. The number in the parenthesis adjacent to the mark O indicates such a ratio (the average ratio of 3 cases). The mark X in the "correspondence with microarray" column indicates a gene that does not correspond with the microarray results. The mark XX indicates a gene, which exhibits an opposite correlation with the microarray results.

In Tables 11A, 11B, 12A, and 12B, the term "correlation'' is used to mean a correlation between the gene expression level and the recurrence period in 22 cases, or in 31 cases wherein the number of months in which the recurrence of the cancer had occurred was determined. In the case of a significant correlation, O or the r value was indicated, and further, the p value was also indicated.

In Tables 11B and 12B, with regard to genes exhibiting a significant difference in expression levels between 19 cases of the recurrence within 24 months, and 6 cases of no recurrence for 40 months or more (the upper case of the "significant difference between two groups" column in Tables 11 Band 12B) or 4 cases of no recurrence for 58 months or more (the lower case of the "significant difference between two groups'' column in Tables 11B and 12B), p values (Mann-Whitney U test) were shown in the "significant difference between two groups" column.

Primer sequences (sense strand (forward), antisense strand (reverse)) used forthe test are shown in Tables 11A, 11B, 12A, and 12B (SEQ ID NOS: 1 to 88).

The results obtained by analyzing the 9 gene candidates (CNgood) up-regulated in nontumor tissues in the late recurrence group of type C hepatocellular carcinoma cases are shown in Tables 11A and 11 B. Table 11A shows the analysis results obtained by quantitative PCR, which was performed on the cases shown in Table 10A as targets, under the conditions shown in Table 11 A using GAPDH as an internal standard gene.

**Table 11A Results of quantitative PCR of "genes up-regulated in nontumor tissues in late recurrence group of hepatitis C cases"**

| No | Gene | Forward/ reverse | primer sequence (5'-3') | SEQ 1D NO | Annealing temperature | Correspondence with microarray | Correlation |
|---|---|---|---|---|---|---|---|
| 26 | PSMB8 | F | AGACTGTCAGTACTGGGAGC | 1 | 60°C | ○(2.52) | |
| | | R | GTCCAGGACCCTTCTTATCC | 2 | | | |
| 27 | RALGDS | F | GACGTGGGAAGACGTTTCCA | 3 | 60°C | ○(4.13) | ○(p=0.0118) |
| | | R | TGGATGATGCCCGTCTCCTT | 4 | | | |
| 28 | APOL3 | F | AATTGCCCAGGGATGAGGCA | 5 | 60°C | ○(2.69) | |
| | | R | TGGACTCCTGGATCTTCCTC | 6 | | | |
| 29 | GBP1 | F | GAGAACTCAGCTGCAGTGCA | 7 | 65°C | ○(6.00) | ○(p=0.0031) |
| | | R | TTCTAGCTGGGCCGCTAACT | 8 | | | |
| 30 | RPS14 | F | GACGTGCAGAAATGGCACCT | 9 | 60°C | × (0.96) | |
| | | R | CAGTCACACGGCAGATGGTT | 10 | | | |
| 31 | CXCL9 | F | CCTGCATCAGCACCAACCAA | 11 | 65°C | ○(11.5) | |
| | | R | TGGCTGACCTGTTTCTCCCA | 12 | | | |
| 32 | DKFZp564F212 | F | CCACATCCACCACTAGACAC | 13 | 60°C | ○(4.75) | ○(p=0.0541) |
| | | R | TGACAGATGTCCTCTGAGGC | 14 | | | |
| 33 | CYP1B1 | F | CCTCTTCACCAGGTATCCTG | 15 | 60°C | ○(2.33) | |
| | | R | CCACAGTGTCCTTGGGAATG | 16 | | | |
| 34 | TNFSF10 | F | GCTGAAGCAGATGCAGGACA | 17 | 60°C | ○(2.50) | ○p=0.0424) |
| | | R | CTAACGAGCTGACGGAGTTG | 18 | | | |

| | | | | | | | |
|---|---|---|---|---|---|---|---|
| With regard to "correspondence with microarray." the ratio of late recurrence group and early recurrence group was obtained from the results of quantitative PCR performed on 6 cases used in microarray analysis, and genes with the ratio of 1.5 or greater were indicated with O. | | | | | | | |
| With regard to "correlation," genes exhibiting correlation between the gene expression levels of 22 cases and the period of time required for recurrence were indicated with O. and the p values thereof were also shown. | | | | | | | |

As a result, it was found that 8 genes corresponded with the microarray results, and that among such genes, 4 genes (RALGDS, GBP1, DKFZp564F212, and TNFSF10) exhibited a correlation with the recurrence period.

Likewise, Table 11B shows the analysis results obtained by quantitative PCR, which was performed on the 10 genes shown in Table 11B and the cases shown in Table 10B as targets, under the conditions shown in the table using G.APDH or 18S rRNA as an internal standard gene.

**Table 11B Results of quantitative PCR of "genes up-regulated in nontumor tissues in late recurrence group of hepatitis C cases"**

| No. | Gene | Forward/reverse | Primer sequence (5'-3') | SEQ ID NO. | Annealing temperature | Correspondence with microarray normalized with GAPDGH | Correspondence with microarray normalized with 185 rRNA | Correlation (GAPDH) | Correlation (185 rRNA) | Significant difference between two groups (GAPDH) | Significant difference betwenn two groups (185 rRNA) |
|---|---|---|---|---|---|---|---|---|---|---|---|
| 1 | M10098 | F | GGAGGTTCGAAGACGATCAG | 19 | 65°C | × × (0.60) | - | | | | |
| | | R | GTGGTGCCCTTCCGTCAATT | 20 | | | | | | | |
| 2 | PSMB8 | F | AGACTGTCAGTACTGGGAGC | 21 | 60°C | ○(1.92) | ○(3.60) | | r=0.421 | | |
| | | R | GTCCAGGACCCTTCTTATCC | 22 | | | | | (p=0.0177) | | |
| 3 | RALGDS | F | GTGTGGCCAACTGTGTCATC | 23 | 65°C | ○(6.71) | ○(8.23) | | r=0.377 | | |
| | | R | CTTCAGACGGTGGATGGAGT | 24 | | | | | (p=0.0361) | 0.0314 | |
| 4 | APOL3 | F | AATTGCCCAGGGATGAGGCA | 25 | 60°C | ○(1.65) | ○(2.13) | | | | |
| | | R | TGGACTCCTGGATCTTCCTC | 26 | | | | | | | |
| 5 | GBP1 | F | AACAAGCTGGCTGGAAAGAA | 27 | 65°C | ○(6.87) | ○(5.76) | r=0.359 | r=0.374 | | |
| | | R | GTACAGGAAGGTGCTGCTCA | 28 | | | | (p=0.0469) | (p=0.0377) | | |
| 6 | RPS14 | F | GACGTGCAGAAATGGCACCT | 29 | 60°C | ○(2.02) | ○(3.35) | r=0.383 | r=0.458 | | 0.0357 |
| | | R | CAGTCACACGGCAGATGGTT | 30 | | | | (p=0.0329) | (p=0.0089) | | |
| 7 | CXCL9 | F | CCTGCATCAGCACCAACCAA | 31 | 65°C | ○(14.3) | ○(12.5) | r=0.392 | r=0.437 | | 0.0131 |
| | | R | TGGCTGACCTGTTTCTCCCA | 32 | | | | (p=0.0282) | (p=0.0132) | | |
| 8 | DKFZp564F212 | F | TGGGCAAGTGAGGTCTTCTT | 33 | 60°C | ○(4.69) | ○(8.40) | | r=0.501 | 0.0485 | 0.0075 |
| | | R | CTGAGGATCACTGGTATCGC | 34 | | | | | (p=0.0036) | 0.0094 | 0.0074 |
| 9 | GYP1B1 | F | GACCCCCAGTCTCAATCTCA | 35 | 65°C | ○(4.29) | ○(4.78) | r=0.424 | r=0.553 | 0.0417 | 0.0042 |
| | | R | AGTCTCTTGGCGTCGTCAGT | 36 | | | | (p=0.0167) | (p=0.001) | 0.0045 | 0.0094 |
| 10 | TNFSF10 | F | GCTGAAGCAGATGCAGGACA | 37 | 60°C | ○(3.71) | ○(4.54) | r=0.460 | r=0.603 | | 0.0062 |
| | | R | CTAACGAGCTGACGGAGTTG | 38 | | | | (p=0.0085) | (p=0.0002) | | 0.0426 |
| | GAPDH | F | GGTCGGAGTCAACGGATTTG | 39 | 60°C | | | | | | |
| | | R | GGATCTCGCTCCTGGAAGAT | 40 | | | | | | | |

| | | | | | | | | | | | |
|---|---|---|---|---|---|---|---|---|---|---|---|
| The expression level of each gene was evaluated by quantitative PCR using GAPDH as a control gene and was expressed as a relative value to the expression level of the control gene. | | | | | | | | | | | |
| With regard to "correspondence with microarray." the ratio of the late recurrence group and the early recurrence group was obtained from the results of quantitative PCR on 6 cases used for microarray analysis. and genes with the ratio of 1.5 or greater were indicated with ○. | | | | | | | | | | | |
| With regard to "correlation." genes exhibiting a correlation between the gene expression levels of 31 cases wherein the number of months of recurrence had been determined. and the period required for recurrence. were indicated with the r value and the p value. | | | | | | | | | | | |
| In "significant difference between two groups." with regard to genes exhibiting a significant difference in expression levels between 19 cases of the recurrence within 24 months. and 6 cases of no recurrence for 40 months or more (the upper case) or 4 cases of no recurrence for 58 months or more (the lower case). p values were indicated (Mann-Whitney U test). | | | | | | | | | | | |

As a result, it was found that when GAPDH was used as an internal standard gene, all the 9 gene candidates exhibiting up-regulation in the late recurrence group corresponded with the microarray results, and that among such genes, 5 genes exhibited a correlation with the recurrence period. In addition, when 18S rRNA was used as an internal standard gene also, all the above 9 gene candidates corresponded with the microarray results, and among them, 8 genes exhibited a correlation with the recurrence period.

A significant difference test was carried out on two groups, the late recurrence group and the early recurrence group. As a result, it was found that when GAPDH was used as a standard gene, 3.genes exhibited a significant difference, and that when 18S rRNA was used as a standard gene, 5 genes exhibited a significant difference.

Subsequently, the results obtained by analyzing the 12 gene candidates (CNbad) up-regulated in nontumor tissues in the early recurrence group of type C hepatocellular carcinoma cases are shown in Tables 12A and 12B. Table 12A shows the analysis results obtained by quantitative PCR, which was performed on the cases shown in Table 10A as targets, under the conditions shown in Table 12A using GAPDH as an internal standard gene.

**Table 12A Results of quantitative PCR of "genes up-regulated in nontumor tissues in early recurrence group of hepatitis C cases"**

| No. | Gene | F/R | Primer sequence (5'-3') | SEQ ID NO. | Annealing temperature | Correspondence with microarray | Correlation |
|---|---|---|---|---|---|---|---|
| 292 | ALB | F R | CAAAGCATGGGCAGTAGCTC CAAGCAGATCTCCATGGCAG | 41 42 | 60°C | ○(2.19) | |
| 293 | NROB2 | F R | TCTTCAACCCCGATGTGCCA AGGCTGGTCGGAATGGACTT | 43 44 | 60°C | ○(1.48) | |
| 267 | AKR1B10 | F R | CTTGGAAGTCTCCTCTTGGC ATGAACAGGTCCTCCCGCTT | 45 46 | 60°C | ○(2.44) | |
| 294 | MAFB | F R | ACCATCATCACCAAGCGTCG TCACCTCGTCCTTGGTGAAG | 47 48 | 60°C | ○(1.56) | |
| 295 | BF530535 | F R | GTCGCCTCACCATCTGTACA CTGGAGGACAGCTGCCAATA | 49 50 | 65°C | ○(3.74) | |
| 296 | MRPL24 | F R | TCCTAGAAGGCAAGGATGCC GTGGGTTTCCTGTCCATAGG | 51 52 | 60°C | × (0.92) | |
| 297 | DSIPI | F R | AACAGGCCATGGATCTGGTG AGGACTGGAACTTCTCCAGC | 53 54 | 65°C | ○(1.85) | |
| 279 | QPRT | F R | AGGATAACCATGTGGTGGCC TGCAGCTCCTCTGGCTTGAA | 55 56 | 60°C | × × (0.413) | ○(p=0.0092) |
| 298 | VNN1 | F R | GCTGGAACTTCAACAGGGAC CTGAGGATCACTGGTATCGC | 57 58 | 60°C | × (1.11) | |
| 299 | IRS2 | F R | TGAAGCTCAACTGCGAGCAG ACGATTGGCTCTTACTGCGC | 59 60 | 60°C | ○(1.57) | |
| 300 | FM05 | F R | ACACAGAGCTCTGAGTCAGC TCCAGGTTAGGAGGGAAGAC | 61 62 | 60°C | × (1.13) | |
| 301 | DCN | F R | CCTCAAGGTCTTCCTCCTTC CACCAGGTACTCTGGTAAGC | 63 64 | 60°C | × (0.74) | |

| | | | | | | | |
|---|---|---|---|---|---|---|---|
| QPRT gene is a gene exhibiting an opposite correlation. | | | | | | | |

As a result, 7 genes corresponded with the microarray results. No genes significantly exhibited a correlation with the recurrence period. However, the QPRT gene significantly exhibited an opposite correlation. Accordingly, this gene was identified as a gene up-regulated in non tumor tissues in the late recurrence group.

Likewise, Table 12B shows the analysis results obtained by quantitative PCR, which was performed on the cases shown in Table 10B as targets, under the conditions shown in Table 12B using GAPDH or 18S rRNA as an internal standard gene.

**Table 12B Results of quantitative PCR of "genes up-regulated in nontumor tissues in early recurrence group of hepatitis C cases"**

| No. | Gene | Forwer/reverse | Primer sequence (5'-3') | SEQ ID NO. | Annealing temperature | Correspondence with microarray. normalized with GAPDH | Correspondence with microarray. normalized with 185 rRNA | Correlation (GAPDH) | Correlation (185 rRNA) | Significant difference between two groups (GAPDH) | Significant difference between two groups (185 rRNA) |
|---|---|---|---|---|---|---|---|---|---|---|---|
| 1 | ALB | F | CAAAGCATGGGCAGTAGCTC | 65 | 60°C | × (1.25) | × × (0.64) | | | | |
| | | R | CAAGCAGATCTCCATGGCAG | 66 | | | | | | | |
| 2 | NR0B2 | F | TCTTCAACCCCGATGTGCCA | 67 | 65°C | × (1.13) | × (1.04) | | | | 0.0220 |
| | | R | AGGCTGGTCGGAATGGACTT | 68 | | | | | | | |
| 3 | AKR1B10 | F | CTTGGAAGTCTCCTCTTGGC | 69 | 60°C | × (0.83) | × (0.92) | | | | |
| | | R | CGCCATCCAGTACAGATCCT | 70 | | | | | | | |
| 4 | MAFB | F | GACGTGAAGAAGGAGCCACT | 71 | 60°C | × (0.71) | × × (0.61) | r=0.422 | r=0.501 | | 0.0281 |
| | | R | CGCCATCCAGTACAGATCCT | 72 | | | | (p=0.0171) | (p=0.036) | | |
| 5 | BF530535 | F | TGCCATAGTGGCTTGATTTG | 73 | 60°C | × (0.82) | × × (4.48) | | | | 0.0486 |
| | | R | TCAGAATCCCCATCATCACA | 74 | | | | | | | |
| 6 | MRPL24 | F | CAGGGCAAAGTGGTTCAAGT | 75 | 65°C | × × (0.46) | × × (0.31) | r=0.431 | r=0.483 | 0.0083 | 0.0083 |
| | | R | TCTCAGTGGGTTTCCTGTCC | 76 | | | | (p=0.0147) | (p=0.0053) | 0.0040 | 0.0426 |
| 7 | DSIPI | F | AACAGGCCATGGATCTGGTG | 77 | 65°C | ○ (2.57) | ○(1.75) | | | | |
| | | R | AGGACTGGAACTTCTCCAGC | 78 | | | | | | | |
| 8 | QPRT | F | AACTACGCAGCCTTGGTCAG | 79 | 65°C | × (0.72) | × × (0.54) | | | | 0.0075 |
| | | R | TGGCAGTTGAGTTGGGTAAA | 80 | | | | | | | 0.0231 |
| 9 | VNN1 | F | GCTGGAACTTCAACAGGGAC | 81 | 65°C | × × (0.65) | × × (0.41) | | | 0.0018 | 0.0009 |
| | | R | CTGAGGATCACTGGTATCGC | 82 | | | | | | 0.0035 | 0.0074 |
| 10 | IRS2 | F | CCACTCGGACAGCTTCTTCT | 83 | 65°C | × (0.78) | × × (0.63) | r=0.419 | r=0.462 | | |
| | | R | GGATGGTCTCGTGGATGTTC | 84 | | | | (p=0.0181) | (p=0.0082) | | |
| 11 | FMO5 | F | ACACAGAGCTCTGAGTCAGC | 85 | 60°C | × (1.02) | × × (0.62) | | | | |
| | | R | TCGAGGTTAGGAGGGAAGAC | 86 | | | | | | | |
| 12 | DCN | F | CCTCAAGGTCTTCCTCCTTC | 87 | 60°C | × (1.40) | × (0.77) | | | | |
| | | R | CACCAGGTACTCTGGTAAGC | 88 | | | | | | | |

| | | | | | | | | | | | |
|---|---|---|---|---|---|---|---|---|---|---|---|
| With regard to "correspondence with microarray." the ratio of the late recurrence group and the early recurrence group was obtained from the results of quantitative PCR of 6 cases used for microarray analysis. and genes with the ratio of 1.5 or greater were indicated with ○. | | | | | | | | | | | |
| × indicates no difference. and × × indicates an opposite correlation. | | | | | | | | | | | |
| With regard to "correlation." genes exhibiting a correlation between the gene expression levels of 31 cases wherein the number of months of recurrence has been determined. and the period required for recurrence. were indicated with the r value (opposite correlation) and the p values. | | | | | | | | | | | |
| With regard to "significant difference between two groups." gene exhibiting a significant difference in expression levels between 19 cases of the recurrence within 24 months. and 6 cases of no recurrence for 40 months or more (the upper case) or 4 cases of no recurrence for 58 months or more (the low case). p values (Mann-Whitney U test) were indicated. | | | | | | | | | | | |

As a result, it was found that when GAPDH or 18S rRNA was used as an internal standard gene, among 12 gene candidates exhibiting up-regulation in the early recurrence group, 1 gene corresponded with the microarray results. However, when GAPDH was used as an internal standard gene, the MAFB gene, the MRPL24 gene, the VNN1 gene, and IRS2 gene significantly exhibited an opposite correlation. In addition, when 18S rRNA was used as an internal standard gene, the NR0B2 gene, the MAFB gene, the BF530535 gene, the MRPL24 gene, the QPRT gene, the VNN1 gene, and the IRS2 gene significantly exhibited an opposite correlation. Accordingly, these genes were identified as genes up-regulated in nontumor tissues in the late recurrence group.

As stated above, as a result of the studies carried out under various conditions, the following 15 genes were identified as genes expressed in nontumor tissues, which can be used for prediction of the recurrence of cancer in type C hepatocellular carcinoma cases: the PSMB8 gene, the RALGDS gene, the GBP1 gene, the RPS14 gene, the CXCL9 gene, the DKFZp564F212 gene, the CYP1B1 gene, the TNFSF10 gene, the NROB2 gene, the MAFB gene, the BF530535 gene, the MRPL24 gene, the QPRT gene, the VNN1 gene, and the IRS2 gene. The meanings of the aforementioned genes are as follows:
PSMB8 gene (which is also referred to as LMP7 gene): A proteasome subunit, beta type, 8 gene
RALGDS gene: A ral guanine nucleotide dissociation stimulator gene
GBP1 gene: A guanylate-binding protein 1 gene
RPS14 gene: A ribosomal protein S14 gene
CXCL9 gene: A chemokine (C-X-C motif) ligand 9 gene
DKFZp564F212 gene: An expression gene discovered by German Human Genome Project, whose gene product has not been identified and whose functions have not yet been predicted.
CYP1B1 gene: A cytochrome P450, family 1, subfamily B, polypeptide 1 gene
TNFSF10: An abbreviation of TNF (ligand) super family, member 10, and a TNF-related apoptosis inducing ligand (TRAIL) gene
NROB2 gene: A nuclear receptor subfamily 0, group B, member 2 gene
MAFB gene: A v-maf musculoaponeurotic fibrosarcoma oncogene homolog B gene
BF530535 gene: A gene whose gene product has not been identified and whose functions have not yet been predicted.
MRPL24 gene: A mitochondrial ribosomal protein L24 gene
QPRT gene: A quinolinate phosphoribosyltransferase gene
VNN1 gene: A vanin 1 gene
IRS2 gene: An insulin receptor substrate 2 gene

### Example 3

Study of correlation between the recurrence period and an expression level of genes in each group in type B hepatocellular carcinoma cases

As mentioned below, with regard to genes up-regulated in the nontumor tissues of a late recurrence group and an early recurrence group in type B hepatocellular carcinoma cases, the correlation between the recurrence period and an expression level was studied.

The total 16 nontumor tissue samples, including 4 cases of type B hepatocellular carcinoma used in the gene expression profile analysis, were used as targets. The clinicopathological findings of each case and the recurrence period (that is, the period of time in which the cancer has not yet recurred) are shown in Table 13.

**Table 13 Type B hepatocellular carcinoma cases**

| Case No. | Sex | Age | Nontumor tissue | stage | Number of months without recurrence | Microarray |
|---|---|---|---|---|---|---|
| 67 | M | 45 | CH | II | >99 | Late recurrence group |
| 87 | M | 45 | CH | I | >92 | |
| 85 | F | 64 | NL | II | 84 | |
| 93 | M | 58 | CH | I | >67 | |
| 94 | F | 59 | LC | I | >66 | |
| 60 | M | 60 | NL | I | 64 | Late recurrence group |
| 35 | M | 69 | CH | I | >48 | |
| 45 | M | 68 | CH | I | >48 | |
| 84 | M | 51 | CH | I/II | 47 | |
| 54 (86) | M | 52 | CH | II | 27 | |
| 47 | M | 36 | CH | I | 23 | |
| 8 | M | 68 | CH | II | 17 | |
| 13 | F | 51 | CH | I | 14 | Early recurrence group |
| 42 (88) | M | 74 | CH | II | 14 | |
| 89 | M | 45 | CH | II | 9 | |
| 9 | M | 44 | CH | II | 7 | Early recurrence group |

| | | | | | | |
|---|---|---|---|---|---|---|
| CH: chronic hepatitis; LC: liver cirrhosis; NL; normal liver | | | | | | |
| The term "stage I/II" indicates that it is unknown whether the stage is stage I or 11. | | | | | | |
| The term "number of months without recurrence" includes not only the number of months required for recurrence, but also includes the investigation period in which recurrence was not observed. | | | | | | |

With regard to the total 71 genes consisting of 24 genes (BNgood) up-regulated in the nontumor tissues of the late recurrence group shown in Table 1 and 47 genes (BNbad) up-regulated in the nontumor tissues of the early recurrence group shown in Table 5, the relationship between the recurrence period and an expression level was analyzed.

First, total RNA was extracted from the nontumor hepatic tissue of each case by the same method as that described in Example 1 above.

In order to eliminate the influence of DNA mixed therein, the total RNA was treated with DNase I (DNase I, TAKARA SHUZO, Kyoto, Japan) at 37°C for 20 minutes, and it was then purified again with a TRIzol reagent. Using 10 µg of the total RNA, a reverse transcription reaction was carried out with 100 µl of a reaction solution comprising 25 units of AMV reverse transcriptase XL (TAKARA) and 250 pmol of a 9-mer random primer.

Real-time PCR was carried out using 0.25 to 50 ng each of synthetic cDNA. 25 µl of a reaction solution, SYBR Green PCR Master mix (Applied Biosystems, Foster City, CA) was used, and ABI PRISM 7000 (Applied Biosystems) was employed. PCR was carried out under conditions wherein preliminary heating was carried out at 95°C for 10 minutes, and thereafter, a cycle consisting of 95°C for 15 seconds and 60°C (or 65°C) for 60 seconds, was repeated 40 to 45 times.

Using GAPDH or 18S rRNA as an internal standard gene of each sample, absolute quantitative analysis was carried out. Values obtained by subjecting standard samples to serial dilution and simultaneous measurement, were used to produce a calibration curve.

The absolute expression level of a target gene and that of an internal standard gene were obtained. Thereafter, the ratio of the target gene expression level/the internal standard gene expression level was calculated for each sample, and it was used for evaluation. All such measurements were carried out in a duplicate manner.

As with the descriptions in Example 2, the term "correspondence with microarray'' shown in Tables 14 and 15 is used to mean that when the ratio of the late recurrence group (case Nos. 67 and 60) and the early recurrence group (case Nos. 13 and 9) was obtained from the results of quantitative PCR performed on 4 cases (case Nos. 67, 60, 13, and 9 in Table 13) used in the microarray analysis, genes, the above ratio of which was 1.5 or greater, corresponded with the results of the microarray in Example 1. The mark O is given to genes, when the above ratio of is 1.5 or greater, and preferably 2 or greater. The number in the parenthesis adjacent to the mark O indicates the value of such a ratio. The mark X in the "correspondence with microarray'' column indicates a gene that does not correspond with the microarray results. The mark XX indicates a gene that exhibits an opposite correlation to the microarray results.

In the "correlation" columns in Tables 14 and 15, with regard to genes, which exhibited a correlation between the gene expression level and the recurrence period in 10 cases wherein the number of months in which the recurrence of the cancer had occurred was determined, the r value and the p value were described.

In the "significant difference between two groups" column in Tables 14 and 15, with regard to genes exhibiting a significant difference in expression levels between 6 cases of the recurrence within 24 months, and 8 cases of no recurrence for 48 months or more (the upper case of the "significant difference between two groups" in Tables 14 and 15) or 6 cases of no recurrence for 60 months or more (the lower case of the "significant difference between two groups" in Tables 14 and 15), p values (Mann-Whitney U test) were indicated.

Primer sequences (sense strand (forward), antisense strand (reverse)) used for the test are shown in Tables 14 and 15 (SEQ ID NOS: 89 to 228).

The results obtained by analyzing the 24 gene candidates (BNgood) up-regulated in nontumor tissues in the late recurrence group of type B hepatocellular carcinoma cases are shown in Tables 14. Table 14 shows the analysis results obtained by quantitative PCR, which was performed on the cases shown in Table 13 as targets, under the conditions shown in Table 14 using GAPDH or 18S rRNA as an internal standard gene.

**Table 14 Results of quantitative PCR of "genes up-regulated in nontumor tissues in late recurrence group of hepatitis B cases"**

| No. | Gene | Forward/reverse | Primer sequence (5'-3') | SEQ ID NO. | Annealing temperature | Correspondence with microarray. noramized with GAPDH | Correspondence with microarray. normalized with 185 rRNA | Correlation (GAPDH) | Correlation (185 rRNA) | Significant difference between two groups (GAPDH) | Significant difference between two groups (185 rRBA) |
|---|---|---|---|---|---|---|---|---|---|---|---|
| 1 | TNFSF14 | F | CTGTTGGTCAGCCAGCAGT | 89 | 65°C | ○(6.11) | ○(2.36) | | | | 0.0065 |
| | | R | GAAAGCCCCGAAGTAAGACC | 90 | | | | | | | |
| 2 | MMP2 | F | CAAGGACCGGTTCATTTGGC | 91 | 60°C | ○(3.82) | ○(2.09) | | | | |
| | | R | GAACACAGCCTTCTCCTCCT | 92 | | | | | | | |
| 3 | SSA2 | F | TGCTCGGGGGAACTATGATG | 93 | 60°C | ○(5.20) | ○(2.47) | | | | |
| | | R | GGCCTGTGAGTCTCTGGATA | 94 | | | | | | | |
| 4 | COL1A1 | F | GGAAGAGTGGAGAGTACTGG | 95 | 60°C | ○(2.56) | × (1.33) | | | | |
| | | R | ATCCATCGGTCATGCTCTCG | 96 | | | | | | | |
| 5 | COL1A2 | F | GTATTCCTGGCCCTGTTGGT | 97 | 60°C | ○(2.92) | ○(1.52) | | | | |
| | | R | CTCACCCTTGTTACCGCTCT | 98 | | | | | | | |
| 6 | DPYSL3 | F | CTTTGAAGGGATGGAGCTGC | 99 | 65°C | ○(1.52) | × (0.78) | | | | |
| | | R | ATCGTACATGCCCCTTGGGA | 100 | | | | | | | |
| 7 | PPARD | F | GGCCTCTATCGTCAACAAGG | 101 | 60°C | × (1.04) | × × (0.40) | | | | |
| | | R | GCGTTGAACTTGACAGCAAA | 102 | | | | | | | |
| 8 | LUM | F | TACCAATGGTGCCTCCTGGA | 103 | 60°C | × (1.38) | × (0.82) | | | | |
| | | R | CCACAGACTCTGTCAGGTTG | 104 | | | | | | | |
| 9 | MSTP032(RGS5) | F | CTGGAAAGGGCCAAGGAGAT | 105 | 60°C | ○(1.79) | × (1.03) | | | | |
| | | R | TCTGGGTCTTGGCTGGTTTC | 106 | | | | | | | |
| 10 | CRP | F | TGGCCAGACAGACATGTCGA | 107 | 60°C | ○(3.43) | ○1.60) | | | | |
| | | R | TCGAGGACAGTTCCGTGTAG | 108 | | | | | | | |
| 11 | TRIM38 | F | TCTCTGGAGGCTGGAGAAAG | 109 | 65°C | × (1.18) | × × (0.49) | | | | |
| | | R | GTTTCCAGCTTCACAGCCCA | 110 | | | | | | | |
| 12 | S100A6 | F | ATTGGCTCGAAGCTGCAGGA | 111 | 60°C | ○(1.83) | × (0.87) | | | | |
| | | R | CGAAGGTGACATACTCCTGG | 112 | | | | | | | |
| 13 | PZP | F | TACTCCAATGCAACCACCAA | 113 | 65°C | ○(4.39) | ○(2.15) | | r=0.717 | | |
| | | R | AACACAAGTTGGGATGCACA | 114 | | | | | (p=0.0171) | | |
| 14 | EMP1 | F | TGGTGTGCTGGCTGTGCATT | 115 | 60°C | ○(1.65) | × (0.92) | | | | |
| | | R | GACCAGATAGAGAACGCCGA | 116 | | | | | | | |
| 15 | AI590053 | F | GTGAATGCCTCTGGAGTGGT | 117 | 65°C | × (1.20) | × × (0.46) | | | | |
| | (AL137672) | R | TTCTGTTCTGACGCCAAGTG | 118 | | | | | | | |
| 16 | MAPIK5 | F | GTTCTAGCCAGTACTTCCGG | 119 | 60°C | ○(1.64) | × (0.69) | | | 0.0528 | |
| | | R | ACTCGCTCCGAATTCTTGC | 120 | | | | | | | |
| 17 | TIM1 | F | ATTCCGACCTCGTCATCAGG | 121 | 60°C | ○(2.91) | ○(1.62) | | | | |
| | | R | GCTGGTATAAGGTGGTCTGG | 122 | | | | | | | |
| 18 | GSTM1 | F | GGACTTTCCCAATCTGCCCT | 123 | 60°C | ○(3.19) | ○(1.64) | | | | |
| | | R | AGGTTGTGCTTGCGGGCAAT | 124 | | | | | | | |
| 19 | CSDA | F | AGGAGAGAAGGGTGCAGAAG | 125 | 60°C | ○(2.50) | × (1.09) | | | | |
| | | R | CCTTCCATAGTAGCCACGTC | 126 | | | | | | | |
| 20 | GSTM2 | F | ACAACCTGTGCGGGGAATCA | 127 | 65°C | ○(1.82) | × (0.75) | | | | |
| | | R | GGTCATAGCAGAGTTTGGCC | 128 | | | | | | | |
| 21 | SGK | F | GGTCATAGCAGAGTTTGGCC | 129 | 60°C | ○(1.75) | × (0.71) | | | | |
| | | R | CAGGCTCTTCGGTAAACTCG | 130 | | | | | | | |
| 22 | LMNA | F | ATGGAGATGATCCCTTGCTG | 131 | 60°C | × (1.11) | × × (0.50) | | | | 0.0282 (opposite) |
| | | R | AGGTGTTCTGTGCCTTCCAC | 132 | | | | | | | 0.0547 (opposite) |
| 23 | MGP | F | GCTCTAAGCCTGTCCACGAG | 133 | 60°C | ○(3.12) | ○(1.63) | | | | |
| | | R | CGCTTCCTGAAGTAGCGATT | 134 | | | | | | | |
| 24 | LTBP2 | F | GCGACACAGGAGTGTCAAGA | 135 | 60°C | ○(2.20) | × (1.21) | | | | |
| | | R | TGACCATGATGTAGCCCTGA | 136 | | | | | | | |

| | | | | | | | | | | | |
|---|---|---|---|---|---|---|---|---|---|---|---|
| With regard to "correspondence with microarray." the ratio of the late recurrence group and the early recurrence group was obtained from the results of quantitative PCR on 4 cases used for microarray analysis. and genes with the ratio of 1.5 or greater were indicated with ○. | | | | | | | | | | | |
| × indicates no difference and × × indicates an opposite correlation. | | | | | | | | | | | |
| With regard to "correlation." genes exhibiting a correlation between the gene expression levels of 10 cases wherein the number of months of recurrence has been determined. and the period required for recurrence. were indicated with the r value (opposite correlation) and the p values. | | | | | | | | | | | |
| In "significant difference between two groups." with regard to gene exhibiting a significant difference in expression levels between 6 cases of the recurrence within 24 months. and 8 cases of no recurrence for 48 months or more (the upper case) or 6 cases of no recurrence for 60 months or more (the low case). p values (Mann-Whitney U test) were indicated. | | | | | | | | | | | |

As a result, it was found that when GAPDH was used as an internal standard gene, 19 out of the 24 gene candidates exhibiting up-regulation in the late recurrence group corresponded with the microarray results, and that among such genes, no genes exhibited a correlation with the recurrence period. In addition, when 18S rRNA was used as an internal standard gene, 9 out of the above 24 gene candidates corresponded with the microarray results, and among them, only 1 gene (PZP gene) exhibited a correlation with the recurrence period

A significant difference test was carried out on two groups, the late recurrence group and the early recurrence group. As a result, it was found that when GAPDH was used as a standard gene, only one gene (MAP3K5 gene) exhibited a significant difference, and that when 18S rRNA was used as a standard gene, only one gene (TNFSF14 gene) exhibited a significant difference. On the contrary, there was one gene (LMNA gene), which had a significant difference, oppositely correlating to the recurrence period. Accordingly, this gene was identified as a gene up-regulated in nontumor tissues in the early recurrence group.

Subsequently, the results obtained by analyzing the 47 gene candidates (BNbad) up-regulated in nontumor tissues in the early recurrence group of type B hepatocellular carcinoma cases are shown in Table 15. Table 15 shows the analysis results obtained by quantitative PCR, which was performed on the cases shown in Table 13 as targets, under the conditions shown in Table 15 using GAPDH or 18S rRNA as an internal standard gene.

**Table 15 Results of quantitative PCR of "genes up-regulated in nontumor tissues in early recurrence group of hepatitis B cases"**

| No. | Gene | Forward/reverse | Primer sequene (5'-3') | SED ID NO. | Annealing temperature | Correspondence with microarray. normalized with GAPDH | Correspondence with microarray. normalized with 185 rRNA | Correlation (GAPDH) | Correlation (185 rRNA) | Significant difference between two groups (GAPDH) | Significant difference between two groups (185 rRNA) |
|---|---|---|---|---|---|---|---|---|---|---|---|
| 1 | CTH | F | TGAATGGCCACAGTGATGTT | 137 | 60°C | ○(4.47) | ○(13.25) | | | | |
| | | R | CCATTCCGTTTTTGAAATGC | 138 | | | | | | | |
| 2 | OAT | F | TCGTAAGTGGGGCTATACCG | 139 | 60°C | ○(2.70) | ○(11.89) | | | | |
| | | R | CTGGTTGGGTCTGTGGAACT | 140 | | | | | | | |
| 3 | PRODH | F | CTGACCACCGGGTGTACTTT | 141 | 60°C | ○(4.61) | ○(22.30) | | | | |
| | | R | GACAAGTAGGGCAGCACCTC | 142 | | | | | | | |
| 4 | CYP3A7 | F | GGAACCCGTACACATGGACT | 143 | 60°C | × × (0.39) | × (1.27) | | | | |
| | | R | AACGTCCAATAGCCCTTACG | 144 | | | | | | | |
| 5 | DDT | F | CGCCCACTTCTTTGAGTTTC | 145 | 60°C | × (1.04) | ○(4.42) | | | | |
| | | R | CATGACCGTCCCTATCTTGC | 146 | | | | | | | |
| 6 | PGRMC1 | F | TATGGGGTCTTTGCTGGAAG | 147 | 65°C | × (1.15) | ○(3.48) | | | | |
| | | R | GCCCACGTGATGATACTTGA | 148 | | | | | | | |
| 7 | AKR1C1 | F | GGTCACTTCATGCCTGTCCT | 149 | 60°C | × (1.32) | ○(3.95) | | | | |
| | | R | TATGGCGGAAGCCAGCTTCA | 150 | | | | | | | |
| 8 | HGD | F | CACCAGCCCTTTGAATCCAT | 151 | 60°C | ○(1.61) | ○(5.80) | | | | |
| | | R | TGTCTCCAGCTCCACACAAG | 152 | | | | | | | |
| 9 | FHR4 | F | TTGAGAATTCCAGAGCCAAGA | 153 | 60°C | × (0.83) | ○(1.85) | | | | |
| | | R | CACCCATCTTCACCACACAC | 154 | | | | | | | |
| 10 | FST | F | AAGACGGAACTGAGCAAGGA | 155 | 60°C | ○(3.58) | ○(6.80) | | | | |
| | | R | TTTTTCCCAGGTCCACAGTC | 156 | | | | | | | |
| 11 | COX4 | F | - | | - | - | - | | | | |
| | | R | - | | | | | | | | |
| 12 | APP | F | CGGGCAAGACTTTTCTTTGA | 157 | 60°C | × (1.28) | ○(4.13) | | | | |
| | | R | TGCCTTCCTCATCCCCTTAT | 158 | | | | | | | |
| 13 | PSPHL | F | TCCAAGGATGATCTCCCACT | 159 | 60°C | ○(4.97) | ○(5.44) | | | | |
| | | R | AGCATCCGATTCCTTCTTCA | 160 | | | | | | | |
| 14 | CYP1A1 | F | TGATAAGCACGTTGCAGGAG | 161 | 65°C | ○(2.77) | ○(11.30) | | | | 0.0389 |
| | | R | AAGTCAGCTGGGTTTCCAGA | 162 | | | | | | | 0.0547 |
| 15 | ZNF216 | F | GGTGTCAGAGCCAGTTGTCA | 163 | 60°C | ○(1.84) | ○(5.39) | | | | |
| | | R | AAATTTCCACATCGGCAGTC | 164 | | | | | | | |
| 16 | LEPR | F | CCACCATTGGTACCATTTCC | 165 | 60°C | ○(5.78) | ○(14.99) | | | | |
| | | R | CCCCTCACCTGAACCTCATA | 166 | | | | | | | |
| 17 | TOM1L1 | F | TTTTCTGGAACATTCAAATTCA | 167 | 60°C | × (0.89) | ○(2.61) | | | | |
| | | R | CACTTTTTGTCATCGCTGGA | 168 | | | | | | | |
| 18 | PECR | F | TGCAGTGGAATACGGATCAA | 169 | 60°C | × (1.19) | ○(3.49) | | | | |
| | | R | GGAAGCAGACCACAGAGGAG | 170 | | | | | | | |
| 19 | ALDH7A1 | F | AGTGGAAGGTGTGGGTGAAG | 171 | 65°C | × (1.34) | ○(3.45) | | | | |
| | | R | CAACCATACACTGCCACAGG | 172 | | | | | | | |
| 20 | GNMT | F | CACTTAAGGAGCGCTGGAAC | 173 | 60°C | ○(1.82) | ○(6.15) | | | | |
| | | R | TTTGCAGTCTGGCAAGTGAG | 174 | | | | | | | |
| 21 | OATPC | F | GCCACTTCTGCTTCTGTGTTT | 175 | 60°C | × (1.27) | ○(3.50) | | | | |
| | | R | TCCACCATAAAAGATGTGGAAA | 175 | | | | | | | |
| 22 | AKR1B10 | F | CCTCCACTCATGTCCCATTT | 177 | 60°C | ○(2.92) | ○(8.05) | | | | |
| | | R | TCAAGCCATGCTTTTCTGTG | 178 | | | | | | | |
| 23 | ANGPTL3 | F | ATTTTAGCCAATGGCCTCCT | 179 | 60°C | × (1.18) | ○(3.37) | | | | |
| | | R | CACTGGTTTGCAGCGATAGA | 180 | | | | | | | |
| 24 | AASS | F | ATTGGTGAATTGGGATTGGA | 181 | 60°C | ○(2.04) | ○(6.83) | | | | |
| | | R | GAAGCCCACCACAGTAGGAA | 182 | | | | | | | |
| 25 | CALR | F | TGGATCGAATCCAAACACAA | 183 | 60°C | × (1.12) | ○(2.77) | | | | |
| | | R | CTGGCTTGTCTGCAAACCTT | 184 | | | | | | | |
| 26 | BAAT | F | CTCCATCATCCACCCACTTT | 185 | 60°C | × (1.15) | ○(4.06) | | | | |
| | | R | GGAAGGCCAGCAAGTGTAGA | 186 | | | | | | | |
| 27 | PMM1 | F | GCCAGAAAATTGACCCTGAG | 187 | 60°C | × (1.04) | ○(3.53) | | | | |
| | | R | CAGCTGCTCAGCGATCTTAC | 188 | | | | | | | |
| 28 | RABR | F | CCCTCATCGTGTCAAGTCAA | 189 | 60°C | × (1.15) | ○(3.78) | | | | |
| | | R | AGGATCAAACAGACCCAACC | 190 | | | | | | | |
| 29 | GLUL | F | TTGTTTGGCTGGGATAGAGG | 191 | 60°C | × (0.85) | ○(2.41) | | | | |
| | | R | GCTCTGTCCGGATAGCTACG | 192 | | | | | | | |
| 30 | CSHMT | F | CCCTACAAGGTGAACCCAGA | 193 | 60°C | × (1.20) | ○(3.33) | | | | |
| | | R | GGAGTAGGAGCTGGTCCGTG | 194 | | | | | | | |
| 31 | UGT1A3 | F | TGACAACCTATGCCATTTCG | 195 | 60°C | × (0.89) | ○ (3.10) | | | | |
| | | R | CCACACAAGACCTATGATAGA | 196 | | | | | | | |
| 32 | HSPG1 | F | CTCAAGGATGACGTGGGTTT | 197 | 60°C | × (1.45) | ○ (4.17) | | | | |
| | | R | GATTTCCTCTGGCCAATTCA | 198 | | | | | | | |
| 33 | QPRT | F | AACTACGCAGCCTTGGTCAG | 199 | 60°C | × (1.24) | ○ (3.91) | | | | |
| | | R | TGGCAGTTGAGTTGGGTAAA | 200 | | | | | | | |
| 34 | DEPP | F | GATGTTACCAATCCCGTTCG | 201 | 60°C | ○ (2.68) | ○ (6.92) | | | | |
| | | R | TGGGCTCCTATATGCGGTTA | 202 | | | | | | | |
| 35 | CA2 | F | TGCTTTCAACGTGGAGTTTG | 203 | 60°C | ○ (1.73) | ○ (4.89) | | | | |
| | | R | CCCCATATTTGGTGTTCCAG | 204 | | | | | | | |
| 36 | FTHFD | F | CAAAATGCTGCTGGTGAAGA | 205 | 60°C | × (1.28) | ○ (4.65) | | | | |
| | | R | GCCTCTGTCAGCTCAAGGAC | 206 | | | | | | | |
| 37 | LAMP1 | F | GTCGTCAGCAGCCATGTTTA | 207 | 60°C | × × (0.61) | ○ (1.97) | | | | |
| | | R | GGCAGGTCAAAGGTCATGTT | 208 | | | | | | | |
| 38 | FKBP1A | F | GGGATGCTTGAAGATGGAAA | 209 | 60°C | × (0.79) | ○ (1.79) | | | | |
| | | R | CAGTGGCACCATAGGCATAA | 210 | | | | | | | |
| 39 | BNIP3 | F | GCTCCTGGGTAGAACTGCAC | 211 | 60°C | × (1.00) | ○ (2.70) | | | | |
| | | R | GCCCTGTTGGTATCTTGTGG | 212 | | | | | | | |
| 40 | MAP3K12 | F | TTGAGGAAATCCTGGACCTG | 213 | 60°C | × × (0.59) | ○ (1.52) | | | | |
| | | R | TTGAGGTCTCGCACCTTCTT | 214 | | | | | | | |
| 41 | ASS | F | CTGATGGAGTACGCAAAGCA | 215 | 60°C | ○ (2.81) | ○ (9.16) | | | | |
| | | R | CTCGAGAATGTCAGGGGTGT | 216 | | | | | | | |
| 42 | ACTB | F | ACAGAGCCTCGCCTTTGC | 217 | 60°C | × (0.74) | ○ (2.04) | | | | |
| | | R | CACGATGGAGGGGAAGAC | 218 | | | | | | | |
| 43 | PLAB | F | GAGCTGGGAAGATTCGAACA | 219 | 60°C | ○ (2.57) | ○ (5.03) | | | | |
| | | R | AGAGATACGCAGGTGCAGGT | 220 | | | | | | | |
| 44 | ENO1L1 | F | GAGATCTCGCCGGCTTTAC | 221 | 60°C | × (0.75) | ○ (2.14) | | | | |
| | | R | CGCGAGAGTCAAAGATCTCC | 222 | | | | | | | |
| 45 | IGFBP3 | F | CAGCTCCAGGAAATGCTAGTG | 223 | 60°C | × (0.86) | ○ (2.81) | | | 0.0528 ( 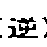 ) | |
| | | R | GGTGGAACTTGGGATCAGAC | 224 | | | | | | | |
| 46 | UK114 | F | GAGGGAAGGCTTAGCCATGT | 225 | 60°C | × (1.11) | ○ (3.13) | | | | |
| | | R | TTGAAGGGTCCATGCCTATC | 226 | | | | | | | |
| 47 | ARF1 | F | GCCTGTAAGTACGGGGACAA | 227 | 60°C | × (1.16) | ○ (2.82) | | | | |
| | | R | CTCTTCAGCGTTGTGGATGA | 228 | | | | | | | |

| | | | | | | | | | | | |
|---|---|---|---|---|---|---|---|---|---|---|---|
| Altough Gene Nos. 22 and 33 are genes common with CNbad, different sequences were used as PCR primers for Gene No. 22. PCR was carried out on Gene No. 11 using 2 primer sets. However, since stable amplification did not achieved in any case, it was pending. Whit regard to "correspondence with microarray," the ratio of the early recurrence group and the late recurrence group was obtained from the results of quantitative PCR on 4 cases used for microarray analysis, and genes with the ratio of 1.5 or greater were indicated with ○. × indicates no difference, and × × indicates an opposite correlation. There were no genes, which exhibites a correlation between the gene expression levels of 10 cases, wherein the number of months of recurrence had been determined, and the period required for recurrence. In "significant difference between two groups," with regard to genes exhibiting a significant difference in expression levels between 6 cases of the recurrence within 24 months, and 8 cases of no recurrence for 48 months or more (the upper case) or 6 cases of no recurrence for 60 months of more (the lower case), p values (Mann-Whitney U test) were indicated. | | | | | | | | | | | |

As a result, it was found that when GAPDH was used as an internal standard gene, 16 gene corresponded with the microarray results, but that no genes significantly exhibited a correlation with the recurrence period. However, the IGFBP3 gene significantly exhibited an opposite correlation in the significant difference test between two groups. Accordingly, this gene was identified as a gene up-regulated in nontumor tissues in the late recurrence group.

In addition, when 18S rRNA was used as an internal standard gene, 45 genes corresponded with the microarray results, but that no genes significantly exhibited a correlation with the recurrence period. However, the CYP1A1 gene significantly exhibited a correlation in a significant difference test between two groups. Accordingly, this gene was identified as a gene up-regulated in nontumor tissues in the early recurrence group.

As stated above, the following 6 genes were identified as genes expressed in nontumor tissues, which can be used for prediction of the recurrence of cancer in type B hepatocellular carcinoma cases: the PZP gene, the MAP3K5 gene, the TNFSF14 gene, the LMNA gene, the CYP1A1 gene, and the IGFBP3 gene. The meanings of the aforementioned genes are as follows:
PZP gene: A pregnancy-zone protein gene
MAP3K5 gene: A mitogen-activated protein kinase kinase kinase 5 gene
TNFSF14 gene: A tumor necrosis factor (ligand) superfamily, member 14 gene
LMNA gene: A lamin A/C gene
CYP1A1 gene: A cytochrome P450, family 1, subfamily A, polypeptide 1 gene
IGFBP3 gene: An insulin-like growth factor binding protein 3 gene

### Example 4

Selection of combination of genes used for distinguishing early recurrence group from late recurrence group

By combining several genes expressed in nontumor tissues used for prediction of the recurrence of type C or B hepatocellular carcinoma, which were obtained from the results of Examples 2 and 3, it becomes possible to carry out recurrence prediction more precisely. As such gene sets, many types of sets are conceived. Examples of the aforementioned combination are shown in Table 16.

**Table 16 Examples of combinations of genes used for distinguishing hepatocellular carcinoma early recurrence group from late recurrence**

| Causal cancer | Early group | Late group | Normalization with GAPDH | Normalization with 18S rRNA |
|---|---|---|---|---|
| Type C hepatocellular cancer | < 24 months | > 40 months | VNN1 MRPL24 | VNN1 CXCL9 GBP1 RALGDS |
| | Classification rate | | 88% | 100% |
| Type B hepatocellular cancer | < 24 months | > 48 months | PRODH LMNA MAP3K12 | LMNA LTBP2 COL1A2 PZP |
| | Classification rate | | 100% | 100% |

### (1) Prediction of type C hepatocellular carcinoma

When GAPDH is used as an internal standard gene for normalization of gene expression in the distinction of an early recurrence group wherein the cancer has recurred within 24 months from a late recurrence group wherein the cancer has not recurred for 40 months or more, the gene expression level of VNN1 and that of MRPL24 may be examined. Otherwise, when 18S rRNA is used as an internal standard gene for normalization in the above distinction, the expression level of each gene of a gene set consisting of VNN1, CXCL9, GBP1, and RALGDS may be examined. The expression level of each of the aforementioned genes is assigned to a discriminant using a discriminant function coefficient obtained regarding each gene, and the obtained value is used for distinction. The expression level of the above gene group is analyzed. In the case of GAPDH normalization, the classification rate between the early recurrence group and the late recurrence group is found to be 88%, and in the case of 18S rRNA, the classification rate is found to be 100%.

### (2) Prediction of type B hepatocellular carcinoma

When GAPDH is used as an internal standard gene for normalization in the distinction of an early recurrence group wherein the cancer has recurred within 24 months from a late recurrence group wherein the cancer has not recurred for 48 months or more, the expression level of each gene of a gene set consisting of PRODH, LMNA, and MAP3K12 may be examined. Otherwise, when 18S rRNA is used as an internal standard gene for normalization in the above distinction, the expression level of each gene of a gene set consisting of LMNA, LTBP2, COL1A2, and PZP may be examined. As described above, such expression levels are assigned to a discriminant, and the obtained values are used for distinction. The expression level of the above gene group is analyzed. In both cases of correlation with GAPDH and 18S rRNA, the classification rate between the early recurrence group and the late recurrence group is found to be 100%.

The meanings of the aforementioned genes are as follows:
PRODH gene: A proline dehydrogenase (oxidase) 1 gene
LTBP2 gene: A latent transforming growth factor beta binding protein 2 gene
COL1A2 gene: A collagen, type I, alpha 1 gene
MAP3K12 gene: A mitogen-activated protein kinase kinase kinase 12 gene

### INDUSTRIAL APPLICABILITY

By identifying common genes derived from a patient and a healthy subject and cause-specific genes, it becomes possible to predict prognosis and recurrence. Accordingly, the thus identified genes can be used for diagnosis, the development of treatment methods, and a strategy of selecting a therapeutic agent (Taylor-made medicine).

### Sequence Listing Free Text

SEQ ID NOS: 1 to 228: synthetic DNA

## Claims

1. A method for evaluating cancer, which comprises the following steps of:
(a) collecting total RNA from an analyte;
(b) measuring the expression level of at least one gene selected from among the genes shown in Tables 1 to 8; and
(c) evaluating cancer using the measurement result as an indicator.

2. A method for evaluating cancer, which comprises the following steps of:
(a) collecting total RNA from an analyte;
(b) measuring the expression level of at least one gene selected from the group consisting of the PSMB8 gene, the RALGDS gene, the GBP1 gene, the RPS14 gene, the CXCL9 gene, the DKFZp564F212 gene, the CYP1B1 gene, the TNFSF10 gene, the NR0B2 gene, the MAFB gene, the BF530535 gene, the MRPL24 gene, the QPRT gene, the VNN1 gene, and the IRS2 gene; and
(c) evaluating cancer using the measurement result as an indicator.

3. A method for evaluating cancer, which comprises the following steps of:
(a) collecting total RNA from an analyte;
(b) measuring the expression level of at least one gene selected from the group consisting of the PZP gene, the MAP3K5 gene, the TNFSF14 gene, the LMNA gene, the CYP1A 1 gene, and the IGFBP3 gene; and
(c) evaluating cancer using the measurement result as an indicator.

4. A method for evaluating cancer, which comprises the following steps of:
(a) collecting total RNA from an analyte;
(b) measuring the expression level of each gene contained in a gene set consisting of the VNN 1 gene and the MRPL24 gene, or a gene set consisting of the PRODH gene, the LMNA gene, and the MAP3K12 gene, using GAPDH as an internal standard gene; and
(c) evaluating cancer using the measurement result as an indicator.

5. A method for evaluating cancer, which comprises the following steps of:
(a) collecting total RNA from an analyte;
(b) measuring the expression level of each gene contained in a gene set consisting of the VNN1 gene, the CXCL9 gene, the GBP1 gene, and the RALGDS gene, or a gene set consisting of the LMNA gene, the LTBP2 gene, the COL1A2 gene, and the PZP gene, using 18S rRNA as an internal standard gene; and
(c) evaluating cancer using the measurement result as an indicator.

6. The method according to any one of claims 1 to 5, wherein the evaluation of cancer involves prediction of the presence or absence of metastasis or recurrence.

7. The method according to any one of claims 1 to 5, wherein the cancer is hepatocellular carcinoma.

8. The method according to claim 2 or 3, wherein the expression level of a gene can be measured by amplifying the gene, using at least one set of primers consisting of the nucleotide sequences shown in SEQ ID NOS: 2n-1 and 2n (wherein n represents an integer between 1 and 114).

9. The method according to claim 4 or 5, wherein the expression level of a gene can be measured by amplifying the gene, using a set of primers for amplifying each gene contained in at least one gene set selected from the group consisting of a gene set consisting of the VNN1 gene and the MRPL24 gene, a gene set consisting of the PRODH gene, the LMNA gene, and the MAP3K12 gene, a gene set consisting of the VNN1 gene, the CXCL9 gene, the GBP1 gene, and the RALGDS gene, and a gene set consisting of the LMNA gene, the LTBP2 gene, the COLIA2 gene, and the PZP gene.

10. A primer set, which comprises at least one set of primers consisting of the nucleotide sequences shown in SEQ ID NOS: 2n-1 and 2n (wherein n represents an integer between 1 and 114).

11. A primer set, which comprises a set of primers for amplifying each gene contained in at least one gene set selected from the group consisting of a gene set consisting of the VNN1 gene and the MRPL24 gene, a gene set consisting of the PRODH gene, the LMNA gene, and the MAP3K12 gene, a gene set consisting of the VNN1 gene, the CXCL9 gene, the GBP1 gene, and the RALGDS gene, and a gene set consisting of the LMNA gene, the LTBP2 gene, the COL1A2 gene, and the PZP gene.

12. A kit for evaluating cancer, which comprises any gene shown in Tables 1 to 8.

13. A kit for evaluating cancer, which comprises at least one gene selected from the group consisting of the RALGDS gene, the GBP1 gene, the DKFZp564F212 gene, the TNFSF10 gene, and the QPRT gene.

14. A kit for evaluating cancer, which comprises each gene contained in at least one gene set selected from the group consisting of a gene set consisting of the VNN1 gene and the MRPL24 gene, a gene set consisting of the PRODH gene, the LMNA gene, and the MAP3K12 gene, a gene set consisting of the VNN1 gene, the CXCL9 gene, the GBP1 gene, and the RALGDS gene, and a gene set consisting of the LMNA gene, the LTBP2 gene, the COL1A2 gene, and the PZP gene.

15. The kit according to any one of claims 12 to 14, which further comprises the primer set according to claim 10 or 11.
